# EUROPEAN PATENT APPLICATION

(11) **EP 0 979 869 A1**
(43) Date of publication of application: **16.02.2000**
(21) Application number: 98114853.9
(22) Date of filing: 07.08.1998
(51) Int. Cl.: C12N 15/11, A61K 31/70, C07H 21/00

(54) **Short oligonucleotides for the inhibition of VEGF expression**

(71) Applicant: Hoechst Marion Roussel Deutschland GmbH, 65929 Frankfurt am Main (DE)
(72) Inventor: Ulhmann, Eugen, Dr., 61479 Glashütten (DE); Peyman, Anuschirwan, Dr., 65779 Kelkheim (DE); Bitonti, Alan J., Ringoes, New Jersey 08551 (US); Woessner, Richard D., Dr., Lebanon, New Jersey 08833 (US)

(57) **Abstract**

The present invention relates to a short oligonucleotide or a derivative thereof which has a sequence that corresponds to a particular part of a nucleic acid sequence which encodes VEGF (vascular endothelial growth factor) and which has a length of maximum 15 nucleotides, the invention further relates to a method of making the oligonucleotide and the use thereof.

A short oligonucleotide or a derivative thereof, which has a length of 10 to 15 nucleotides and which corresponds to a part of a VEGF encoding sequence, wherein the part of the VEGF encoding sequence to which the oligonucleotide corresponds to has one of the sequences SEQ ID NO. 1, SEQ ID NO. 2, SEQ ID NO. 3, SEQ ID NO. 4, SEQ ID NO. 5 or SEQ ID NO. 6 or a part thereof,
wherein
SEQ ID NO. 1 is 5'- CCCGGCCCCGGTCGGGCCTCCG - 3',
SEQ ID NO. 2 is 5'- CGGGCCTCCGAAACC -3' ,
SEQ ID NO. 3 is 5'- GCTCTACCTCCACCATGCCAA -3',
SEQ ID NO. 4 is 5'- GTGGTCCCAGGCTGCACCCATGGC -3',
SEQ ID NO. 5 is 5'- CATCTTCAAGCCATCC -3', and
SEQ ID NO. 6 is 5'- TGCGGGGGCTGCTGC -3'.

## Description

The present invention relates to a short oligonucleotide or a derivative thereof which has a sequence that corresponds to a particular pad of a nucleic acid sequence which encodes VEGF (vascular endothelial growth factor) and which has a length of maximum 15 nucleotides, the invention further relates to a method of making the oligonucleotide and the use thereof.

Angiogenesis is defined as the growth of new capillary blood vessels and plays a fundamental role in growth and development. In mature human the ability to initiate an angiogenic response is present in all tissues, but is held under strict control. Angiogenesis is only mobilized in specific situations, such as wound repair and endometrial regulation. The regulation of angiogenesis relies on a fine balance between numerous inhibitory and stimulatory factors. VEGF, also called VPF (vascular permeability factor), is a key regulator of angiogenesis and its mitogenic effect is specific for endothelial cells (Ferrara, Trends Cardiovasc. Med. (1993) 3, 244). VEGF exists in at least four different isoforms (VEGF₁₂₁, VEGF₁₆₅, VEGF₁₈₉ and VEGF₂₀₆) that exert similar biological activities and result from alternative splicing. VEGF is expressed in abnormally high levels in human tumors and in diseased tissues characterized by high degree of vascularization or vascular permeability, such as diabetic retinopathy, psoriasis, age-related macular degeneration, rheumatoid arthritis and other inflammatory diseases. Therefore, agents which selectively decrease the VEGF levels may be used to treat malignancies and other angiogenic diseases.

It has been shown that monoclonal antibodies against VEGF can suppress the growth of several tumors in nude mice (Kim et al., Nature (1993) 362, 841). Another possibility for reducing VEGF levels is the use of antisense oligonucleotides, which are optionally modified in order to improve their properties (E. Uhlmann and A. Peyman, Chemical Reviews 90, 543 (1990); S. Agrawal, TIBTECH 1996, 376). Antisense oligonucleotides are thought to bind to specific sequences of the mRNA resulting in degradation of the mRNA and/or inhibition of protein synthesis.

EP 0769 552 A1 discloses antisense oligonucleotides having a length of 8 nucleotides or longer directed against different regions of the VEGF encoding sequence. Some of these oligonucleotides were shown to inhibit the expression of VEGF to 30% or less. The oligonucleotides were tested in a cell free system in form of unmodified oligonucleotides (with no phosphodiester internucleoside bridge modification). Selected antisense oligonucleotides, ranging in size from 16 to 20 nucleotides, were also tested in form of the all-phosphorothioates (all phosphodiester internucleoside bridges are modified as phosphorothioate) (oligonucleotides A085R-S, A087P-S, A227-S, A287-S, A311-S, and A419-S) showing 30-46 % inhibition of VEGF expression at 20 µM of all-phosphorothioate oligonucleotide in a A549 cell-based assay. The most effective all-phosphorothioate oligonucleotide (A419-S) is a 20-mer and has the sequence SEQ ID NO. 100: 5'-TGGTGAGGTTTGATCCGCAT-3'.

The present invention provides a short oligonucleotide or a derivative thereof, which corresponds to a part of a VEGF encoding sequence,
wherein the part of the VEGF encoding sequence to which the oligonucleotide corresponds to has one of the sequences SEQ ID NO. 1, SEQ ID NO. 2, SEQ ID NO. 3, SEQ ID NO. 4, SEQ ID NO. 5 or SEQ ID NO. 6 or a part thereof, and
wherein
SEQ ID NO. 1 is 5'- CCCGGCCCCGGTCGGGCCTCCG - 3',
SEQ ID NO. 2 is 5'- CGGGCCTCCGAAACC -3',
SEQ ID NO. 3 is 5'- GCTCTACCTCCACCATGCCAA -3',
SEQ ID NO. 4 is 5'- GTGGTCCCAGGCTGCACCCATGGC -3',
SEQ ID NO. 5 is 5'- CATCTTCAAGCCATCC -3', and
SEQ ID NO. 6 is 5'- TGCGGGGGCTGCTGC -3'.

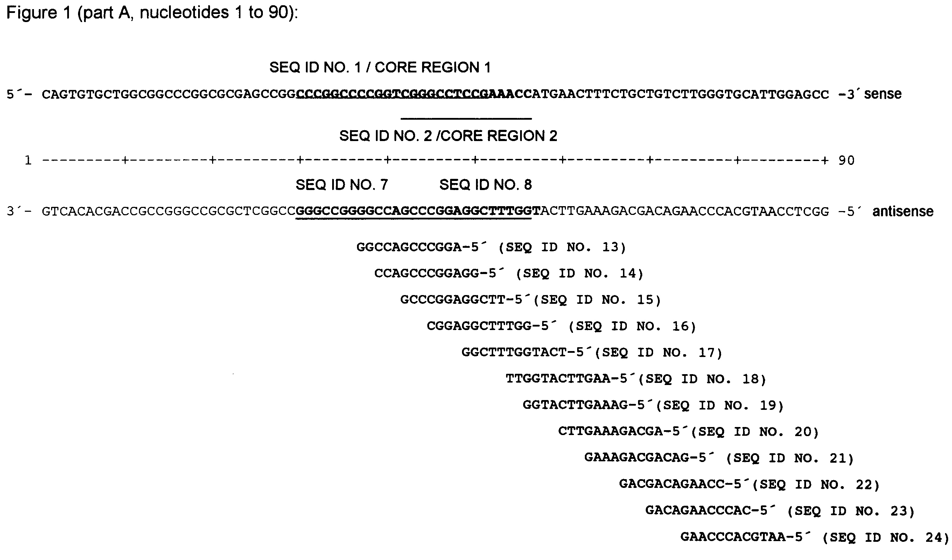

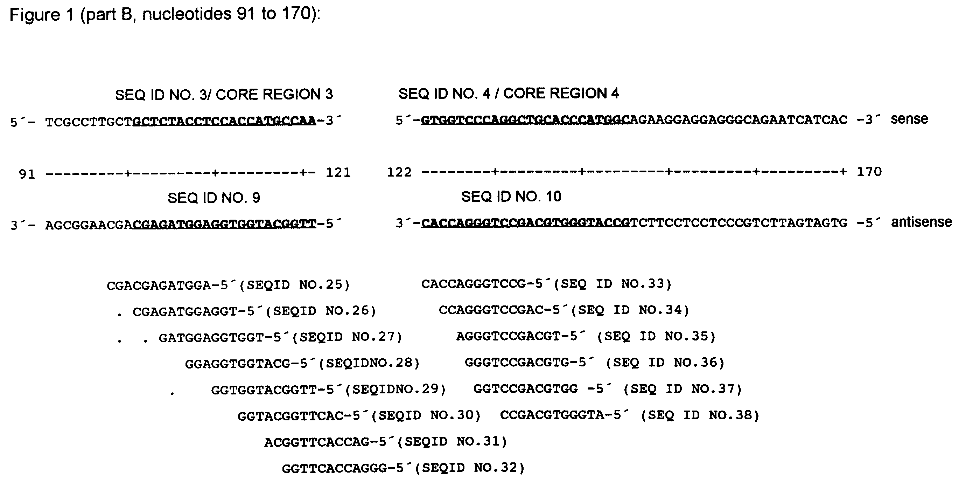

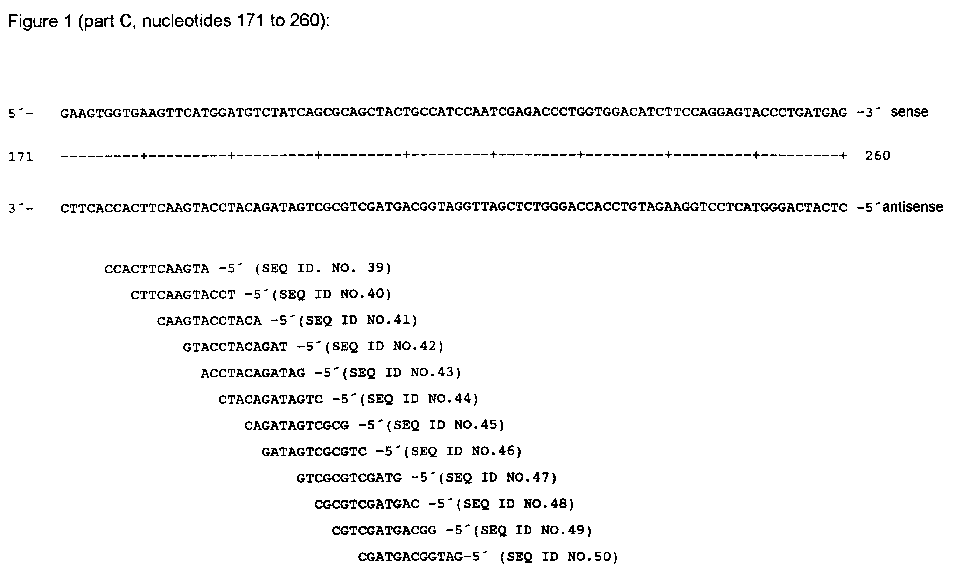

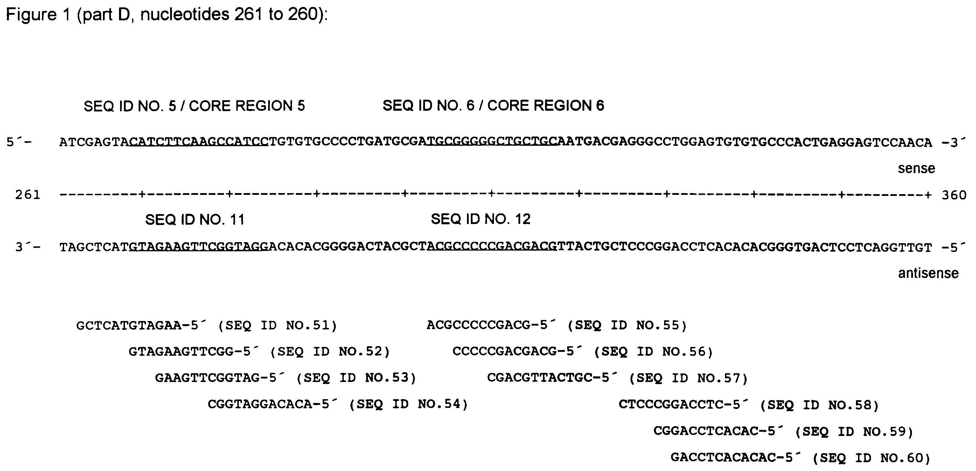

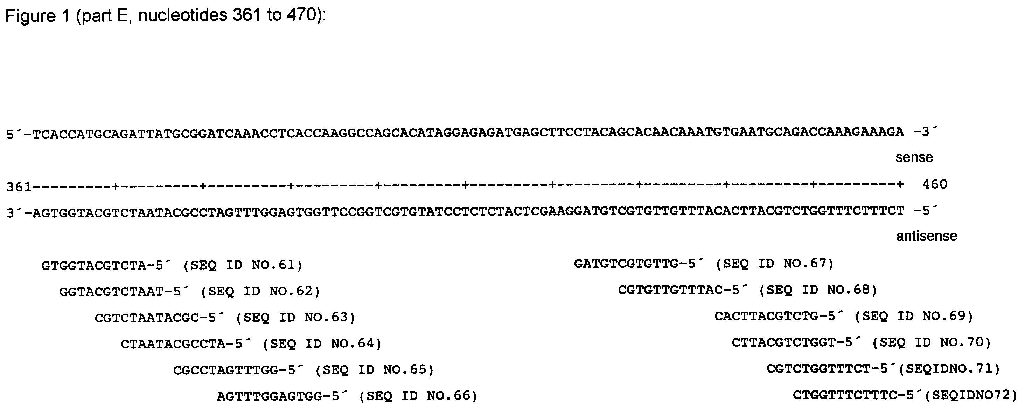

Sequences SEQ ID NO. 1, SEQ ID NO. 2, SEQ ID NO. 3, SEQ ID NO. 4, SEQ ID NO. 5 and SEQ ID NO. 6 are "core regions", which have been identified to be extremely suitable for the design of short oligonucleotides. Sequences SEQ ID NO. 1, SEQ ID NO. 2, SEQ ID NO. 3, SEQ ID NO. 4, SEQ ID NO. 5 and SEQ ID NO. 6 are equivalent to nucleotides 30 to 51 (SEQ ID NO.1 which is named core region 1), nucleotides 42 to 56 (SEQ ID NO. 2 which is named core region 2), nucleotides 101 to 121 (SEQ ID NO. 3 which is named core region 3), nucleotides 122 to 145 (SEQ ID NO. 4 which is named core region 4), nucleotides 268 to 284 (SEQ ID NO. 5 which is named care region 5) and nucleotides 303 to 317 (SEQ ID NO: 6 which is named core region 6). The numbering refers to the human VEGF nucleotide sequence SEQ ID NO. 93 (table 1). The localization of the core sequences within sequence SEQ ID NO: 93 is shown in figure 1. A nucleotide sequence for human VEGF cDNA is given in figure 1 B in Leung et al. (1989) Science 8, 1307. Sequence SEQ ID NO. 93 corresponds to the 5'-end (to nucleotides 1 to 480) of the sequence shown in figure 1B in Leung et al..

The oligonucleotide has a sequence that corresponds to a part of a nucleic acid which encodes VEGF. The phrase "corresponds to" means that the base sequence of the oligonucleotide is complementary to a part of a nucleic acid sequence, that encodes VEGF (e.g. gene, cDNA, mRNA) and therefore allows the oligonucleotide to hybridize to (bind to) that "sense" part of the VEGF encoding nucleic acid (which is preferably a VEGF mRNA). This is why it is called "antisense oligonucleotide". Therefore, in a preferred embodiment of the invention, the oligonucleotide is an antisense oligonucleotide. In another preferred embodiment of the invention the oligonucleotide is a ribozyme. A ribozyme is a catalytic nucleic acid which cleaves mRNA. Preferably the ribozyme is selected from the group of hammerhead ribozymes (Uhlmann and Peyman, 1990).

An oligonucleotide according to the invention is equivalent to one of the sequences SEQ ID NO. 7 to SEQ ID NO. 12 or a part thereof respectively,
wherein
SEQ ID NO. 7 is 3'- GGGCCGGGGCCAGCCCGGAGGC-5' (corresponds to SEQ ID NO. 1),
SEQ ID NO. 8 is 3'- GCCCGGAGGCTTTGG -5' (Corresponds to SEQ ID NO. 2),
SEQ ID NO. 9 is 3'- CGAGATGGAGGTGGTACGGTT -5' (corresponds to SEQ ID NO. 3),
SEQ ID NO. 10 is 3'- CACCAGGGTCCGACGTGGGTACCG -5' (corresponds to SEQ ID NO. 4),
SEQ ID NO. 11 is 3'- GTAGAAGTTCGGTAGG -5' (corresponds to SEQ ID NO. 5), and
SEQ ID NO. 12 is 3'- ACGCCCCCGACGACG -5' (corresponds to SEQ ID NO. 6).

The part of the VEGF encoding nucleic acid sequence to which the oligonucleotide corresponds to has a length of 10, 11, 12, 13, 14 or 15 nucleotides, preferably the oligonucleotide corresponds to a length of 12 nucleotides of a VEGF encoding sequence. Therefore, an oligonucleotide according to the invention has a length of 10 (10mer), 11 (11mer), 12 (12mer), 13 (13mer), 14 (14mer) or 15 nucleotides (15mer).

In a prefered embodiment of the invention, the oligonucleotide has a length of 12 nucleotides; such oligonucleotides might for example have one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56,
wherein
SEQ ID NO. 14 is 3'- CCAGCCCGGAGG -5' (is equivalent to a part of SEQ ID NO. 7),
SEQ ID NO. 16 is 3'- CGGAGGCTTTGG -5' (is equivalent to a pad of SEQ ID NO. 8),
SEQ ID NO. 27 is 3'- GATGGAGGTGGT -5' (is equivalent to a part of SEQ ID NO. 9),
SEQ ID NO. 28 is 3'- GGAGGTGGTACG -5' (is equivalent to a part of SEQ ID NO. 9),
SEQ ID NO. 29 is 3'- GGTGGTACGGTT -5' (is equivalent to a part of SEQ ID NO. 9),
SEQ ID NO. 33 is 3'- CACCAGGGTCCG -5' (is equivalent to a part of SEQ ID NO. 10),
SEQ ID NO. 34 is 3'- CCAGGGTCCGAC -5' (is equivalent to a part of SEQ ID NO. 10),
SEQ ID NO. 35 is 3'-AGGGTCCGACGT -5' (is equivalent to a part of SEQ ID NO. 10),
SEQ ID NO. 36 is 3'- GGGTCCGACGTG -5' (is equivalent to a part of SEQ ID NO. 10),
SEQ ID NO. 37 is 3'- GGTCCGACGTGG -5' (is equivalent to a part of SEQ ID NO. 10),
SEQ ID NO. 38 is 3'- CCGACGTGGGTA -5' (is equivalent to a part of SEQ ID NO. 10),
SEQ ID NO. 52 is 3'- GTAGAAGTTCGG -5' (is equivalent to a part of SEQ ID NO. 11),
SEQ ID NO. 55 is 3'- ACGCCCCCGACG -5' (is equivalent to a part of SEQ ID NO. 12), and
SEQ ID NO. 56 is 3'- CCCCCGACGACG -5' (is equivalent to a part of SEQ ID NO. 12).

In another embodiment of the invention, the oligonucleotide has a length of 13 nucleotides; such oligonucleotide might for example have one of the sequences SEQ ID NO. 73, SEQ ID NO. 74 or SEQ ID NO. 75,
wherein
SEQ ID NO. 73 is 3'- GGAGGTGGTACGG -5' (is equivalent to a part of SEQ ID NO. 9),
SEQ ID NO. 74 is 3'- GGGTCCGACGTGG -5' (is equivalent to a part of SEQ ID NO. 10), and
SEQ ID NO. 75 is 3'- GCCCCCGACGACG -5' (is equivalent to a part of SEQ ID NO. 12).

In another embodiment of the invention, the oligonucleotide has a length of 14 nucleotides; such oligonucleotide might for example have one of the sequences SEQ ID NO. 76, SEQ ID NO. 77, SEQ ID NO. 78 or SEQ ID NO. 79, wherein
SEQ ID NO. 76 is 3'- CCCGGAGGCTTTGG -5' (is equivalent to a part of SEQ ID NO. 8),
SEQ ID NO. 77 is 3'- CGAGATGGAGGTGG -5' (is equivalent to a part of SEQ ID NO. 9),
SEQ ID NO. 78 is 3'- GGGTCCGACGTGGG -5' (is equivalent to a part of SEQ ID NO. 10), and
SEQ ID NO. 79 is 3'- CGCCCCCGACGACG -5' (is equivalent to a part of SEQ ID NO. 12).

In another embodiment of the invention, the oligonucleotide has a length of 15 nucleotides; such oligonucleotide might for example have one of the sequences SEQ ID NO. 80 to SEQ ID NO. 88,
wherein
SEQ ID NO. 80 is 3'- GGGCCGGGGCCAGCC -5' (is equivalent to a part of SEQ ID NO. 10),
SEQ ID NO. 81 is 3'- CCGGGGCCAGCCCGG -5' (is equivalent to a part of SEQ ID NO. 10),
SEQ ID NO. 82 is 3'- GGCCGGGGCCAGCCC -5' (is equivalent to a part of SEQ ID NO. 10),
SEQ ID NO. 83 is 3'- CCCCGGAGGCTTTGG -5' (is equivalent to a part of SEQ ID NO. 10),
SEQ ID NO. 84 is 3'- ATGGAGGTGGTACGG -5', (is equivalent to a part of SEQ ID NO. 10),
SEQ ID NO. 85 is 3'- GGAGGTGGTACGGTT -5', (is equivalent to a part of SEQ ID NO. 10),
SEQ ID NO. 86 is 3'- CCAGGGTCCGACGTG - 5', (is equivalent to a part of SEQ ID NO. 10),
SEQ ID NO. 87 is 3'- GTAGAAGTTCGGTAG -5' (is equivalent to a pad of SEQ ID NO. 10), and
SEQ ID NO. 88 is 3'- TAGAAGTTCGGTAGG -5' (is equivalent to a pad of SEQ ID NO. 10).

The sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 and SEQ ID NO. 73 to SEQ ID NO. 88 correspond to one of the core sequences or a part thereof (they are equivalent to one of the sequences SEQ ID NO. 7 to SEQ ID NO. 12 or a part thereof). For sequences SEQ ID NO. 13, SEQ ID NO. 15, SEQ ID NO. 17 to to SEQ ID NO. 26, SEQ ID NO. 30 to SEQ ID NO. 32, SEQ ID NO. 39 to SEQ ID NO: 51, SEQ ID NO. 53, SEQ ID NO. 54, SEQ ID NO. 57 to SEQ ID NO: 72 the sequences do not correspond to one of the core regions (oligonucleotides in example 1, figure 1). All the sequences are derived from the sequence of human VEGF cDNA SEQ ID NO. 93 or the VEGF sequence, which was e.g. reported by Leung et al. (Science (1989) 246, 1306).

The invention also relates to derivatives of the oligonucleotides, for example their salts, in particular their physiologically tolerated salts. Salts and physiologically tolerated salts are e. g. described in Remingtons Pharmaceuticals Science (1985) Mack Publishing Company, Easton, PA (page 1418). Derivatives also relate to modified oligonucleotides which have one or more modifications (e.g. at particular nucleoside positions and/or at particular internucleoside bridges, oligonucleotide analogues (e.g. Polyamide-Nucleic Acids (PNAs), Phosphonic acid monoester nucleic acids (PHONAs = PMENAs), oligonucleotide chimeras (e.g. consisting of a DNA- and a PNA-part or consisting of a DNA- and a PHONA-part)).

A preferred subject of the invention relates to an oligonucleotide which has a sequence that corresponds to one of the sequences SEQ ID NO. 1 to SEQ ID NO. 6 or a part thereof (a sequence that is equivalent to one of the sequences SEQ ID NO. 7 to SEQ ID NO. 12 or a part thereof), preferably one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 and SEQ ID NO. 73 to SEQ ID NO. 58 and which is modified to a certain extent. Most preferably an oligonucleotide is modified in order to improve its properties, e.g. in order to increase its resistance to nucleases or to make it resistant against nucleases, respectively to improve its binding affinity to a complementary VEGF encoding nucleic acid e.g. mRNA, or in order to increase its cellular uptake.

Therefore, the present invention preferably relates to an oligonucleotide which has a particular sequence as outlined above and which has in addition one or more chemical modifications in comparison to a "natural" DNA, which is composed of the "natural" nucleosides deoxyadenosine (adenine + β-D-2'-deoxyribose), deoxyguanosine (guanine + β-D-2'-deoxyribose), deoxycytidine (cytosine + β-D-2'-deoxyribose) and thymidine (thymine + β-D-2'-deoxyribose ) linked via phosphodiester internucleoside bridges. The oligonucleotide can have one or more modifications of the same type and/or modifications of a different type; each type of modification can independently be selected from the types of modifications known to be used for modifying oligonucleotides.

Examples of chemical modifications are known to the skilled person and are described, for example, in E. Uhlmann and A. Peyman, Chemical Reviews 90 (1990) 543 and "Protocols for Oligonucleotides and Analogs" Synthesis and Properties & Synthesis and Analytical Techniques, S. Agrawal, Ed, Humana Press, Totowa, USA 1993 and S.T. Crooke, F. Bennet, Ann. Rev. Pharmacol. Toxicol. 36 (1996) 107-129; J. Hunziker and C. Leuman (1995) Mod. Synt. Methods, 7, 331-417.

For example, in comparison to natural DNA a phosphodiester internucleoside bridge, a β-D-2'-deoxyribose unit and/or a natural nucleoside base (adenine, guanine, cytosine, thymine) can be modified or replaced, respectively. An oligonucleotide according to the invention can have one or more modifications, wherein each modification is located at the a particular phosphodiester internucleoside bridge and/or at a particular β-D-2'-deoxyribose unit and/or at a particular natural nucleoside base position in comparison to an oligonucleotide of the same sequence which is composed of natural DNA.

For example, the invention relates to an oligonucleotide which comprises one or more modifications and wherein each modification is independently selected from
a) the replacement of a phosphodiester internucleoside bridge located at the 3'-and/or the 5'- end of a nucleoside by a modified internucleoside bridge,
b) the replacement of phosphodiester bridge located at the 3'- and/or the 5'-end of a nucleoside by a dephospho bridge,
c) the replacement of a sugar phosphate unit from the sugar phosphate backbone by another unit,
d) the replacement of a β-D-2'-deoxyribose unit by a modified sugar unit,
e) the replacement of a natural nucleoside base by a modified nucleoside base,
f) the conjugation to a molecule which influences the properties of the oligonucleotide,
g) the conjugation to a 2'5'-linked oligoadenylate or a derivative thereof, optionally via an appropriate linker, and
h) the introduction of a 3'-3' and/or a 5'-5' inversion at the 3' and/or the 5' end of the oligonucleotide.

More detailed examples for the chemical modification of an oligonucleotide are
a) the replacement of a phosphodiester internucleoside bridge located at the 3'-and/or the 5'- end of a nucleoside by a modified internucleoside bridge, wherein the modified internucleoside bridge is for example selected from phosphorothioate, phosphorodithioate, NR¹R^{1'}-phosphoramidate, boranophosphate, phosphate-(C₁-C₂₁)-O-alkyl ester, phosphate-[(C₆-C₁₂)aryl-((C₁-C₂₁) -O-alkyl]ester, (C₁-C₈)alkylphosphonate and/or (C₆-C₁₂)-arylphosphonate bridges, (C₇-C₁₂)-α-hydroxymethylaryl (e.g. disclosed in WO 95/01363), wherein (C₆-C₁₂)aryl, (C₆-C₂₀)aryl and (C₆-C₁₄)aryl are optionally substituted by halogene, alkyl, alkoxy, nitro, cyano, and where R¹ and R^{1'} are, independently of each other, hydrogen, (C₁-C₁₈)-alkyl, (C₆-C₂₀)-aryl, (C₆-C₁₄)-aryl-(C₁-C₈)-alkyl, preferably hydrogen, (C₁-C₈)-alkyl, preferably (C₁-C₄)-alkyl and/or methoxyethyl,
   or
   R¹ and R^{1'} form, together with the nitrogen atom carrying them, a 5-6-membered heterocyclic ring which can additionally contain a further heteroatom from the group O, S and N,
b) the replacement of a phosphodiester bridge located at the 3'- and/or the 5'-end of a nucleoside by a dephospho bridge (dephospho bridges are described, for example, in Uhlmann, E. and Peyman, A. in "Methods in Molecular Biology", Vol. 20, "Protocols for Oligonucleotides and Analogs", S. Agrawal, Ed., Humana Press, Totowa 1993, Chapter 16, 355ff), wherein a dephospho bridge is for example selected from the dephospho bridges formacetal, 3'-thioformacetal, methylhydroxylamine, oxime, methylenedimethyl-hydrazo, dimethylenesulfone and/or silyl groups;
c) the replacement of a sugar phosphate unit (β-D-2'-deoxyribose and phosphodiester internucleoside bridge together form a sugar phosphate unit) from the sugar phosphate backbone (sugar phosphate backbone is composed of sugar phosphate units) by another unit, wherein the other unit is for example suitable to built up a
   - "morpholino-derivative" oligomer (as described, for example, in E.P. Stirchak et at., Nucleic Acids Res. 17 (1989) 6129), that is e.g. the replacement by a morpholino-derivative unit;
   - polyamide nucleic acid ("PNA") (as described for example, in P.E. Nielsen et al., Bioconj. Chem. 5 (1994) 3 and in EP 0672677 A2), that is e.g. the replacement by a PNA backbone unit, e.g. by 2-aminoethylglycine;
   - phosphonic acid monoester nucleic acid ("PHONA") (as described e.g. in Peyman et al., Angew. Chem. Int. Ed. Engl. 35 (1996) 2632-2638 and in EP 0739898 A2), that is e.g. the replacement by a PHONA backbone unit;
d) the replacement of a β-D-2'-deoxyribose unit by a modified sugar unit,
   wherein the modified sugar unit is for example selected from β-D-ribose, α-D-2'-deoxyribose, L-2'-deoxyribose, 2'-F-2'-deoxyribose, 2'-O-(C₁-C₆)alkyl-ribose, preferably 2'-O-(C₁-C₆)alkyl-ribose is 2'-O-methylribose, 2'-O-(C₂-C₆)alkenyl-ribose, 2'-[O-(C₁-C₆)alkyl-O-(C₁-C₆)alkyl]-ribose, 2'-NH₂-2'-deoxyribose, β-D-xylo-furanose, α-arabinofuranose, 2,4-dideoxy-β-D-erythro-hexo-pyranose, and carbocyclic (described, for example, in Froehler, J. Am. Chem. Soc. 114 (1992) 8320) and/or open-chain sugar analogs (described, for example, in Vandendriessche et at., Tetrahedron 49 (1993) 7223) and/or bicyclosugar analogs (described, for example, in M. Tarkov et al., Helv. Chim. Acta 76 (1993) 481);
e) the replacement of a natural nucleoside base by a modified nucleoside base,
   wherein the modified nucleoside base is for example selected from uracil, hypoxanthine, 5-(hydroxymethyl)uracil, N²-Dimethylguanosine, pseudouracil, 5-(hydroxymethyl)uracil, 5-aminouracil, dihydrouracil, 5-fluorouracil, 5-fluorocytosine, 5-chlorouracil, 5-chlorocytosine, 5-bromouracil, 5-bromocytosine, 2,4-diaminopurine, 8-azapurine, a substituted 7-deazapurine, preferably 7-deaza-7-substituted and/or 7-deaza-8-substituted purine or other modifications of a natural nucleoside bases, (modified nucleoside bases are e.g. described in EP 0 710 667 A2 and EP 0 680 969 A2);
f) the conjugation to a molecule which influences the properties of the oligonucleotide, wherein the conjugation of the oligonucleotide to one or more molecules which (favorably) influence the properties of the oligonucleotide (for example the ability of the oligonucleotide to penetrate, the cell membrane or to enter a cell, the stability against nucleases, the affinity for a VEGF encoding target sequence, the pharmacokinetics of the oligonucleotide, the ability of an antisense oligonucleotide/ribozyme or a molecule conjugated to the oligonucleotide respectively to attack the VEGF encoding target sequence, e.g. the ability to bind to and/or to crosslink, when the oligonucleotide hybridizes with the VEGF encoding target sequence), wherein examples for molecules that can be conjugated to an oligonucleotide are polylysine, intercalating agents such as pyrene, acridine, phenazine or phenanthridine, fluorescent agents such as fluorescein, crosslinking agents such as psoralen or azidoproflavin, lipophilic molecules such as (C₁₂-C₂₀)-alkyl, lipids such as 1,2-dihexadecyl-rac-glycerol, steroids such as cholesterol or testosterone, vitamins such as vitamin E, poly- or oligoethylene glycol preferably linked to the oligonucleotide via a phosphate group (e.g. triethylenglycolphosphate, hexaethylenglycolphosphate), (C₁₂-C₁₈)-alkyl phosphate diesters and/or O-CH₂-CH(OH)-O-(C₁₂-C₁₈)-alkyl, these molecules can be conjugated at the 5' end and/or the 3' end and/or within the sequence, e.g. to a nucleoside base in order to generate an oligonucleotide conjugate; processes for preparing an oligonucleotide conjugate are known to the skilled person and are described, for example, in Uhlmann, E. & Peyman, A., Chem. Rev. 90 (1990) 543, M. Manoharan in "Antisense Research and Applications", Crooke and Lebleu, Eds., CRC Press, Boca Raton, 1993, Chapter 17, p. 303ff. and EP-A 0 552 766;
g) the conjugation to a 2'5'-linked oligoadenylate, preferably via an appropriate linker molecule, wherein the 2'5'-linked oligoadenylate is for example selected from 2'5'-linked triadenylate, 2'5'-linked tetraadenylate, 2'5'-linked pentaadenylate, 2'5'-linked hexaadenyltat or 2'5'-linked heptaadenylat molecules and derivatives thereof, wherein a 2'5'-linked oligoadenylate derivative is for example Cordycepin (2'5'-linked 3'-deoxy adenylate) and wherein an example for an appropriate linker is triethylenglycol and wherein the 5'-end of the 2'5'-linked oligoadenylate must bear a phosphate, diphosphate or triphosphate residue in which one or several oxygen atoms can be replaced e.g. by sulfur atoms, wherein the substitution by a phosphate or thiophosphate residue is preferred; and
h) the introduction of a 3'-3' and/or a 5'-5' inversion at the 3' and/or the 5' end of the oligonucleotide, wherein this type of chemical modification is known to the skilled person and is described, for example, in M. Koga et al, J. Org. Chem. 56 (1991) 3757, EP 0 464 638 and EP 0 593 901.

The replacement of a sugar phosphate unit from the sugar phosphate backbone by another unit, which is e.g. a PNA backbone unit or a PHONA backbone unit, is preferably the replacement of a nucleotide by e.g. a PNA unit or a PHONA unit, which already comprise natural nucleoside bases and/or modified nucleoside bases, e.g. one of the modified nucleoside bases from uracil, hypoxanthine, 5-(hydroxymethyl)uracil, N²-Dimethylguanosine, pseudouracil, 5-(hydroxymethyl)uracil, 5-aminouracil, pseudouracil, dihydrouracil, 5-fluorouracil, 5-fluorocytosine, 5-chlorouracil, 5-chlorocytosine, 5-bromouracil, 5-bromocytosine, 2,4-diaminopurine, 8-azapurine, a substituted 7-deazapurine, preferably 7-deaza-7-substituted and/or 7-deaza-8-substituted purine or other modifications of a natural nucleoside bases, (modified nucleoside bases are e.g. described in EP 0 710 667 A2 and EP 0 680 969 A2);

The oligonucleotide modifications described in EP 0 710 667 A2, EP 0 680 969 A2, EP 0 464 638, EP 0 593 901, WO 95/01363, EP 0 672 677 A2, EP 0 739 898 A2 and EP 0 552 766 are hereby incorporated by reference.

In a special embodiment of the invention, one or more phosphodiester internucleoside bridges within the oligonucleotide sequence are modified, preferably one or more phosphodiester internucleoside bridges are replaced by phosphorothioate internucleoside bridges and/or (C₆-C₁₂)aryl phosphonate internucleoside bridges, preferably by α-hydroxybenzyl phosphonate bridges in which the benzyl group is preferably substituted, e.g. with nitro, methyl, halogen. In an all-phosphorothioate oligonucleotide, all phosphodiester internucleoside bridges are modified by phosphorothioate. Preferably, the invention relates to an oligonucleotide in which not all phosphodiester internucleoside bridges are modified uniformly with phosphorothioate (phosphorothioate internucleoside bridges). Preferably, at least one internucleoside bridge has a different type of modification or is not modified. In particular the invention relates to an oligonucleotide which comprises in addition at least one other type of modification.

In another special embodiment of the invention, one or more nucleosides (β-D-2'-deoxyribose and/or nucleoside base) within the oligonucleotide sequence are modified, preferably the β-D-2'-deoxyribose is substituted by 2'-O-(C₁-C₆)alkylribose, preferably by 2'-O-methylribose and/or the nucleoside base is substituted by 8-aza-purine, 7-deaza-7-substituted purine and/or 7-deaza-8-substituted purine (purine: odenine, guanine). Preferably, the invention relates to an oligonucleotide in which not all nucleosides are modified uniformly. Preferably the invention relates to an oligonucleotide which comprises in addition at least one other type of modification.

In another special embodiment of the invention, one or more sugar phosphate units from the sugar-phosphate backbone are replaced by PNA backbone units, preferably by 2-aminoethylglycine units. Preferably the sugar phosphate units which are replaced are connected together at least to a certain extend. Preferably, the invention relates to an oligonucleotide in which not all sugar phosphate units are uniformly replaced. In particular the invention relates to chimeric oligonucleotides, e.g. composed of one or more PNA parts and one or more DNA parts. For such chimeric oligonucleotides, for example the following non-limiting examples of modification patterns are possible: DNA-PNA, PNA-DNA, DNA-PNA-DNA, PNA-DNA-PNA, DNA-PNA-DNA-PNA, PNA-DNA-PNA-DNA. Comparable patterns would be possible for chimeric molecules composed of DNA parts and PHONA parts, e.g. DNA-PHONA, PHONA -DNA, DNA- PHONA -DNA, PHONA -DNA- PHONA, DNA-PHONA -DNA- PHONA, PHONA -DNA- PHONA -DNA. In addition of course, chimeric molecules comprising three different parts like DNA part(s), PHONA part(s) and PNA pad(s) are possible. Preferably the invention relates to an oligonucleotide which comprises in addition at least one other type of modification.

In another special embodiment of the invention, the oligonucleotide is connected at its 3'end and/or at its 5'end to a (C₁₂-C₁₈)alkyl residue, preferably a C₁₆ alkyl residue, a triethylenglycol residue or a hexaethylenglycol residue - these residues are preferably connected to the oligonucleotide via a phosphate group. Preferably, the invention relates to an oligonucleotide in which not both ends (3'and 5'end) are (uniformly) modified. Preferably, the invention relates to an oligonucleotide which comprises in addition at least one other type of modification.

In a preferred embodiment of the invention only particular positions within an oligonucleotide sequence are modified (e.g. partially modified oligonucleotide). Partially modified oligonucleotides are also named minimal modified oligonucleotides in some documents. Within the sequence a modification can be located at particular positions (at particular nucleotides, at particular nucleosides, at particular nucleoside bases, at particular internucleoside bridges).

In a particular embodiment of the invention, a partially modified oligonucleotide is prepared by only replacing some of the phosphodiester bridges with modified internucleoside bridges, e.g. phosphorothioate bridges and/or α-hydroxybenzyl phosphonate bridges. In particular, the invention comprises such oligonucleotides which are only modified to a certain extent.

In particular the invention relates to an oligonucleotide, wherein the terminal 1 to 5 nucleotide units at the 5' end and/or at the 3' end of the oligonucleotide are protected by modifying internucleoside bridges located at the 5'and/or the 3' end of the corresponding nucleosides, preferably by replacement of the phosphodiester internucleoside bridges by phosphorothioate bridges and/or α-hydroxybenzyl phosphonate bridges. Most preferably the terminal 1 to 5 nucleotide units at the 3' end of the oligonucleotide are protected by modifying internucleoside bridges located at the 5'and/or the 3' end of the corresponding nucleosides. Optionally, the terminal 1 to 5 nucleotide units at the 5' end of the oligonucleotide are in addition protected by modifying intemucleoside bridges located at the 5'and/or the 3' end of the corresponding nucleosides. Optionally, the oligonucleotide may comprise additional modifications at other positions.

Furthermore, the invention relates to an oligonucleotide, wherein at least one internal pyrimidine nucleoside and/or an internucleoside bridge located at the 5'end and/or the 3'end of this pyrimidine nucleoside (a nucleoside with a pyrimidine base like cytosine, uracil, thymine) is modified, preferably by replacement of the phosphodiester internucleoside bridge(s) by phosphorothioate bridge(s) and/or α-hydroxybenzyl phosphonate bridge(s).

In a preferred embodiment of the invention the terminal 1 to 5 nucleotide units at the 5' end and/or at the 3' end of the oligonucleotide are protected by modifying internucleoside bridges located at the 5'and/or the 3' end of the corresponding nucleosides and wherein in addition at least one internal pyrimidine nucleoside and/or an internucleoside bridge located at the 5'end of this pyrimidine nucleoside and/or located at the 3'end of this pyrimidine nucleoside is modified.

The principle of partially modified oligonucleotides is described e.g. in A. Peyman, E. Uhlmann, Biol. Chem. Hoppe-Seyler, 377 (1996) 67-70 and in EP 0 653 439. These documents are hereby incorporated by reference. In this case, 1-5 terminal nucleotide units at the 5' end/or and at the 3' end are protected, e.g. the phosphodiester internucleoside bridges located at the 3'and/or the 5'end of the corresponding nucleosides are for example replaced by phosphorothioate internucleoside bridges. In addition, preferably at least one internal pyrimidine nucleoside (or nucleotide respectively) position is modified; preferably the 3' and/or the 5' internucleoside bridge(s) of a pyrimidine nucleoside is/are modified/replaced, for example by phosphorothioate internucleoside bridge(s). Partially modified oligonucleotides exhibit particularly advantageous properties; for example they exhibit a particularly high degree of nuclease stability in association with minimal modification. They also have a significantly reduced propensity for non-antisense effects which are often associated with the use of all-phosphorothioate oligonucleotides (Stein and Krieg (1994) Antisense Res. Dev. 4, 67). Partially modified oligonucleotides also show a higher binding affinity than all-phosphorothioates.

The invention relates in particular to partially/minimally modified oligonucleotides. Examples for such oligonucleotides which have one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 and in which particular internucleoside bridges are modified are ON1, ON4, ON15, ON16, ON17, ON21, ON 22, ON 23, ON 24, ON 25, ON 26, ON 22, ON 39, ON 40, ON 58; and ON 100 to ON 113:
ON 2 3'-C*C*A G C*C*C*G G A G*G -5' (example for SEQ ID NO: 14),
ON 4 3'-C*G*G A G G C*T*T*T G*G -5' (example for SEQ ID NO: 16),
ON 15 3'-G*A*T G G A G G*T*G G*T -5' (example for SEQ ID NO: 27),
ON 16 3'-G*G*A G G*T G G*T A C*G -5' (example for SEQ ID NO: 28),
ON100 3'-G*G*A G G*T*G G*T A C*G -5' (example for SEQ ID NO: 28),
ON101 3'-G*G*A G G*T G G*T A*C*G -5' (example for SEQ ID NO: 28),
ON102 3'-G*G*A G G*T*G G*T A*C*G -5' (example for SEQ ID NO: 28),
ON103 3'-G*G*A G G*T G G*T*A C*G -5' (example for SEQ ID NO: 28),
ON 17 3'-G*G*T*G G T*A C*G G T*T -5' (example for SEQ ID NO: 29),
ON 21 3'-C*A*C*C A G G G T*C*C*G -5' (example for SEQ ID NO: 33),
ON 22 3'-C*C*A G G G T*C*C G A*C -5' (example for SEQ ID NO: 34),
ON 23 3'-A*G*G G T*C*C G A C*G*T -5' (example for SEQ ID NO: 35),
ON24 3'-G*G G*T*C*C G A C*G T*G -5' (example for SEQ ID NO: 36),
ON104 3'-G*G*G T*C*C G A C*G T*G -5' (example for SEQ ID NO: 36),
ON105 3'-G*G*G T*C*C G A C*G*T*G -5' (example for SEQ ID NO: 36),
ON106 3'-G*G*G T*C*C G A C*G*T G -5' (example for SEQ ID NO: 36),
ON107 3'-G*G G*T*C*C G A C*G*T*G -5' (example for SEQ ID NO: 36),
ON108 3'-G*G*G*T*C*C G A C*G T*G -5' (example for SEQ ID NO: 36),
ON 109 3'-G*G*G*T*C*C G A C*G*T*G -5' (example for SEQ ID NO: 36),
ON110 3'-G*G*G T*C*C G A C*G*T*G -5' (example for SEQ ID NO: 36),
ON111 3'-G*G*G T*C*C*G A C*G T*G -5' (example for SEQ ID NO: 36),
ON112 3'-G*G*G*T*C*C G A C*G T*G -5' (example for SEQ ID NO: 36),
ON113 3'-G*G*G*T*C*C G A C*G*T*G -5' (example for SEQ ID NO: 36),
ON 25 3'-G*G*T C*C*G A C*G T*G G -5' (example for SEQ ID NO: 37),
ON 26 3'-C*C*G A*C G*T G G G*T*A -5' (example for SEQ ID NO: 38),
ON 58 3'-G*T*A G A A G*T T*C*G*G -5' (example for SEQ ID NO: 52),
ON 39 3'-A*C*G C*C*C C*C G A C*G -5' (example for SEQ ID NO: 55), and
ON 40 3'-C*C*C*C C*G A*C G A C*G -5' (example for SEQ ID NO: 56),
wherein " * " denotes the localization of a internucleoside bridge modification; preferably " * " is a phosphorothioate internucleoside bridge.

Another example for a special embodiment of the invention relates to a partially modified oligonucleotide which has a modification of a nucleoside, e.g. a modification of a nucleoside base and/or a modification of a β-D-2'-deoxyribose unit. Preferably a β-D-2'-deoxyribose is replaced by 2'-O-(C₁-C₆)alkylribose, most preferred is the replacement by 2'-O-methylribose (replacement of β-D-2'-deoxyribonucleoside by 2'-O-methylribonucleoside). Examples of such oligonucleotides which have e.g. one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 display the following pattern of nucleoside modification shown in oligonucleotides ON114 to ON138 (only the "N" modification, not the "*" internucleoside modification).

According to the invention, the oligonucleotide can have in addition to one type of modification, also other types of modification.

Therefore, in another embodiment of the invention the oligonucleotide comprises modified internucleoside bridges at particular positions and in addition modification of a nucleoside at particular positions, preferably the replacement of β-D-2'-deoxyribose. In a preferred embodiment of the invention, the internucleoside modification is the replacement of a phosphodiester bridge by a phosphorothioate bridge and the modification of the β-D-2'-deoxyribose is the replacement by 2'-O-methylribose; in this case, the oligonucleotide is a chimeric oligonucleotide, which is composed of modified and unmodified DNA and RNA parts - which comprise the 2'-O-methyl-ribonucleosides and β-D-2'-deoxyribonucleosides and phosphorodiester and phosphorothioate internucleoside bridges.

Examples for such oligonucleotides, which have the sequence SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 and modifications at particular internucleoside bridges and in addition at particular nucleoside positions are ON114 to ON138 (examples for patterns of modifications):
ON114 3'-C*C*A G C*C*C*G G A G*G-'5 (example for SEQ ID NO. 14),
ON115 3'-C*G*G A G G C*T*T*T G*G-'5 (example for SEQ ID NO. 16),
ON116 3'-G*A*T G G A G G*T*G G*T-'5 (example for SEQ ID NO. 27),
ON117 3'-G*G*A G G*T G G*T A C*G-'5 (example for SEQ ID NO. 28),
ON118 3'-G*G*T*G G T*A C*G G T*T-'5 (example for SEQ ID NO. 29),
ON119 3'-C*A*C*C A G G G T*C*C*G-'5 (example for SEQ ID NO. 33),
ON120 3'-C*C*A G G G T*C*C G A*C-'5 (example for SEQ ID NO. 34),
ON121 3'-A*G*G G T*C*C G A C*G*T-'5 (example for SEQ ID NO. 35),
ON122 3'-G*G G*T*C*C G A C*G T*G-'5 (example for SEQ ID NO. 36),
ON123 3'-G*G*T C*C*G A C*G T*G G-'5 (example for SEQ ID NO. 37),
ON124 3'-C*C*G A*C G*T G G G*T*A-'5 (example for SEQ ID NO. 38),
ON125 3'-C*C*C*C C*G A*C G A C*G-'5 (example for SEQ ID NO. 56),
ON126 3'-G*G*G T*C*C G A C*G T*G-'5 (example for SEQ ID NO. 36),
ON127 3'-G*G*G T*C*C G A C*G T*G-'5 (example for SEQ ID NO. 36),
ON128 3'-G*G*G T*C*C G A C*G T*G-'5 (example for SEQ ID NO. 36),
ON130 3'-G*G*G T*C*C G A C*G T*G-'5 (example for SEQ ID NO. 36),
ON131 3'-G*G*G T*C*C G A C*G T*G-'5 (example for SEQ ID NO. 36),
ON132 3'-G*G*A G G*T G G*T A C*G-'5 (example for SEQ ID NO. 28),
ON133 3'-G*G*A G G*T G G*T A C*G-'5 (example for SEQ ID NO. 28),
ON134 3'-G*G*A G G*T G G*T A C*G-'5 (example for SEQ ID NO. 28),
ON135 3'-G*G*A G G*T G G*T A C*G-'5 (example for SEQ ID NO. 28),
ON136 3'-G*G*A G G*T G G*T A C*G-'5 (example for SEQ ID NO. 28),
ON137 3'G*G*A G G*T G G*T A C*G-'5 (example for SEQ ID NO. 28), and
ON138 3'-G*G*A G G*T G G*T A C*G-'5 (example for SEQ ID NO. 28),
wherein
" * " shows the position of a internucleoside bridge modification an wherein an underlined "N" is a modified nucleoside (e.g. modification of the nucleoside base and/or modification of the β-D-2'-deoxyribose). Preferably, "*" is a phosphorothioate bridge and "N" indicates the position of a 2'-O-(C₁-C₆)alkylribonucleoside, preferably a 2'-O-methylribonucleoside.

Further examples are oligonucleotides in which each nucleoside is replaced by 2'-O-allkyl-ribonucleosides (totally composed of 2'-O-alkylribonucleosides; 2'-O-alkyl-RNA). Such oligonucleotides might be additionally stabilized against nucleases by partial replacement of phosphodiester internucleoside bridges by phosphoro-thioate bridges:
ON139 3'-C*C*A G C*C*C*G G A G*G-5' (example for SEQ ID NO. 14),
ON140 3'-C*G*G A G G C*T*T*T G*G-5' (example for SEQ ID NO. 16),
ON141 3'-G*A*T G G A G G*T*G G*T-5' (example for SEQ ID NO. 27),
ON142 3'-G*G*A G G*T G G*T A C*G-5' (example for SEQ ID NO. 28),
ON143 3'-G*G*T*G G T*A C*G G T*T-5' (example for SEQ ID NO. 29),
ON144 3'-C*A*C*C A G G G T*C*C*G-5' (example for SEQ ID NO. 33),
ON145 3'-C*C*A G G G T*C*C G A*C-5' (example for SEQ ID NO. 34),
ON146 3'-A*G*G G T*C*C G A C*G*T-5' (example for SEQ ID NO. 35),
ON147 3'-G*G G*T*C*C G A C*G T*G-5' (example for SEQ ID NO. 36),
ON148 3'-G*G*T C*C*G A C*G T*G G-5' (example for SEQ ID NO. 37),
ON149 3'-C*C*G A*C G*T G G G*T*A-5' (example for SEQ ID NO. 38),
ON150 3'-A*C*G C*C*C C*C G A C*G-5' (example for SEQ ID NO. 55),
ON151 3'-C*C*C*C C*G A*C G A C*G-5' (example for SEQ ID NO. 56), and
ON152 3'-G*T*A G A A G*T T*C*G*G-5' (example for SEQ ID NO. 52),
wherein
" * " shows the position of a internucleoside bridge modification and wherein an underlined "N" is a modified nucleoside (e.g. modification of a nucleoside base and/or modification of a β-D-2'-deoxyribose). Preferably, "*" is a phosphorothioate bridge and "N" indicates the position of a 2'-O-alkylribonucleoside, preferably a 2'-O-methylribonucleoside (in this case T is 2'- O-methyluridine).

A further preferred embodiment of the invention provides an oligonucleotide which has one or more (C₁₂-C₁₈)-alkyl residues, preferably a C₁₆-alkyl residue at its 3' and/or its 5' end. A (C₁₂-C₁₈)-alkyl residue can e.g. be bound as a phosphodiester as described in EP 0 552 766 A2 (EP 0 552 766 A2 is hereby incorporated by reference) or as a 3'-phosphodiester of O-CH₂-CH(OH)-O-(C₁₂-C₁₈)-alkyl. Preferred is an oligonucleotide that has a C₁₆-alkyl residue bound to its 3'- and/or 5'-end.

Examples for such oligonucleotides are ON153 to ON164 (having one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 and modifications at particular internucleoside bridges, like e.g. in ON1, ON4, ON15, ON16, ON17, ON21, ON 22, ON 23, ON 24, ON 25, ON 26, ON 22, ON 39, ON 40 and ON 58 and in addition a C₁₆-alkyl residue linked either to its 5' end or to its 3' end) (such oligonucleotides might also have any other sequence and pattern of modification):
ON153 3'-C*C*A G C*C*C*G G A G*G-C16-5',
ON154 3'-C*G*G A G G C*T*T*T G*G-C16-5',
ON155 3'-G*A*T G G A G G*T*G G*T-C16-5',
ON156 3'-G*G*A G G*T G G*T A C*G-C16-5',
ON157 3'G*G*T*G G T*A C*G G T*T-C16-5',
ON158 3'-C*A*C*C A G G G T*C*C*G-C16-5',
ON159 3'-C*C*A G G G T*C*C G A*C-C16-5',
ON160 3'-A*G*G G T*C*C G A C*G*T-C16-5',
ON161 3'-G*G G*T*C*C G A C*G T*G-C16-5',
ON162 3'G*G*T C*C*G A C*G T*G G-C16-5',
ON163 3'-C*C*G A*C G*T G G G*T*A-C16-5', and
ON164 3'-C*C*C*C C*G A*C G A C*G-C16-5',
wherein
" * " shows a position of the internucleoside bridge modification, preferably the localization of a phosphorothioate internucleoside bridge and wherein "-C16" indicates the position of a modification at the 5'-end by hexadecyl phosphate.

The invention also relates to an oligonucleotide, in which the 3'- and/or the 5'end is connected to an oligoethylenglycol residue, preferably a tri-ethylenglycol or a hexaethylenglycol, most preferably via a phosphodiester (tri- or hexa-ethyleneglycol phosphate ester). Of course, such oligonucleotide may also comprise additional modifications. Non limiting examples for such oligonucleotides which have sequence SEQ ID NO. 36 are ON165, ON166 and ON 167:
ON165 3'-teg-G*G*G T*C*C G A C*G T*G -5',
ON166 3'-teg-G*G*G T*C*C G A C*G T*G -5',
ON167 3'-teg-G*G*G T*C*C G A C*G T*G -5',
wherein
"teg" is an oligoethylenglycol residue linked as phosphate ester to the oligonucleotide, preferably "teg" is a triethylenglycole or hexaethylenglycol phosphate ester, " *" shows the position of the internucleoside bridge modification and wherein an underlined "N" is a modified nucleoside (e.g. modification of the nucleoside base and or modification of the β-D-2'-deoxyribose). Preferably, "*" is a phosphorothioate bridge and "N" indicates the position of a 2'-O-alkylribonucleoside, preferably a 2'-O-methylribonucleoside (in this case "T" is 2'-O-methyluridine).

In another specific embodiment of the invention the oligonucleotide is connected via a linker to a 2'5'-linked oligoadenylate-5'-(thio)phosphate. The linker can e.g. be an oligo-ethylenglycol-phosphate, preferably triethylenglycol-phosphate, tetra-ethylenglycol-phosphate or hexa-ethylenglycol-phosphate residue. The 2'5'-linked oligoadenylate is preferably attached via its 2'-end as a tetra- or as a pentaadenylate whose 5'-hydroxy function is substituted by a phosphate or thiophosphate residue. The 2'5'-oligoadenylate is known to induce RNase L to cleave the target mRNA (Torrence et al., Proc. Natl. Acad. Sci. U.S.A. (1993) 90, 1300). The 2'5'-oligoadenylate serves the purpose to activate ribonuclease L (RNase L) which then degrades the VEGF mRNA. Instead of a 2'5'-linked adenylate, e.g. a 2'5'-linked 3'-deoxy adenylate, derived from the nucleoside analog cordycepin, can be introduced. In this case, the oligonucleotide part, which is complementary to the target nucleic acid is preferably modified at particular positions by 2'-O-(C₁-C₆)alkylribonucleoside (preferably 2'-O-methylribonucleoside) or by PNA. An examples for such an oligonucleotide, which has the sequence SEQ ID NO. 36 is ON168 (such oligonucleotide might also have any other sequence of an oligonucleotide according to the invention):
ON168 5'-p*-(2'5'-rA*rA*rA*rA)*(teg)G*TG*CAGC*C*T*GG*G-3'
wherein
"teg" is a oligoethylenglycol residue, preferably a triethyleneglycol residue,
"N" is a β-D-2'deoxyribonucleoside substituted by a 2'-O-alkylresidue, preferably by a 2'-O-CH₃ ("T" is 2'-O-methyluridine),
"rA" is a ribo-A; Co = 3'-deoxy-A (Cordycepin),
"p*" is a 5'-thiophosphate,
"*" is a modified internucleoside bridge, preferably a phosphorothioate internucleoside bridge.

Another preferred embodiment of the invention involves the replacement of one or more natural nucleoside base(s), by non-natural or modified nucleoside bases respectively, preferably by 8-aza-purines and/or 7-deaza-7-substituted purines and/or 7-deaza-8-substituted purine e.g. as described in EP 0 171 066 and EP 0 680 969. Examples for such oligonucleotides are ON 169 and ON 170 (both have sequence SEQ ID NO. 36 and in addition to the nucleoside base modification other types of modification):
ON169 3'-G*G*G T*C*C G A C*G T*G -5', and
ON170 3'-teg-G*G*G T*C*C G A C*G T^{*}G -5',
wherein
" G " is a 8-aza-deoxyguanosine,
" A "is a 8-aza-deoxyadenosine
"teg" is a oligoethylenglycole phosphate ester, preferably a triethylenglycole phosphate ester,
" N " is a 2'-O-alkylribonucleoside, preferably a 2'-O-methylribonucleoside wherein "T" is 2'-O-alkyluridine, preferably 2'-O-methyluridine.

In another preferred embodiment of the invention, the oligonucleotide can exhibit 3'3' and / or 5'5'-inversions at the 3' and / or 5'-end e.g. as described in EP 0 464 635 and EP 0 593 901. An example for such oligonucleotide is ON171, which has the sequence SEQ ID NO. 36 and in addition to the 3'3'inversion at the 3,end also another type of modification:
ON171 3'-G(3'3')G*G T*C*C G A C*G T*G-5',
wherein
" (3'3') " is a 3'3' phosphodiester linkage and
" * " is a modified internucleoside bridge, preferably a phosphorothioate internucleoside bridge.

Another preferred embodiment of the invention relates to the replacement of one or more phosphodiester bridges by α-hydroxybenzyl phosphonate bridges as described in WO 95/01363. An example for such an oligonucleotide is ON172, which has the sequence SEQ ID NO. 36 and in addition to the replacement of a phosphodiester bridge by a α-hydroxybenzyl phosphonate internucleoside bridge the replacement of phosphodiester bridges by phosphorothioate internucleoside bridges:
ON172 3'-G(hbp)G*G T*C*C G A C*G T*G-5',
wherein
"(hbp)" is an α-hydroxybenzyl phosphonate bridge, preferably an α-hydroxy(o-nitrophenyl)methylphosposphonate bridge and
"*" is a phosphorothioate bridge.

In another preferred embodiment of the invention the oligonucleotide comprises a modification of the sugar phosphate backbone, preferably by PNA units. Examples of such PNA-DNA chimeras, which have one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 and SEQ ID NO. 56 may have the following patterns of modifications (pattern:PNA-DNA) (for the synthesis and properties of PNA-DNA chimeras see EP 0 672 677):
ON173 (3')-c c a g c c c G G A G G-5' (example for SEQ ID NO. 14),
ON174 (3')-c g g a g g c T T T G G-5' (example for SEQ ID NO. 16),
ON175 (3')-g a t g g a G G T G G T-5' (example for SEQ ID NO. 27),
ON176 (3')-g g a g g t G G T A C G-5' (example for SEQ ID NO. 28),
ON177 (3')-g g t g g t a C G G T T-5' (example for SEQ ID NO. 29),
ON178 (3')-c a c c a g g G T C C G-5' (example for SEQ ID NO. 33),
ON179 (3)-c c a g g g t C C G A C-5' (example for SEQ ID NO. 34),
ON180 (3')-a g g g t c c G A C G T-5' (example for SEQ ID NO. 35),
ON181 (3')-g g g t c c G A C G T G-5' (example for SEQ ID NO. 36),
ON182 (3')-g g t c c g a C G T G G-5' (example for SEQ ID NO. 37),
ON183 (3')-c c g a c g t G G G T A-5' (example for SEQ ID NO. 38),
ON184 (3')-c c c c c g a C G A C G-5' (example for SEQ ID NO. 56),
wherein
the lower case letters indicate PNA units,
underlined letters indicate hydroxy ethyl glycine-PNA units,
large letters indicate DNA.

Also other patterns of modifications are possible e.g. DNA-PNA-DNA, PNA-DNA. Comparable patterns of modification are also possible for PHONA/DNA chimeras. These modification patterns can be combined with any other type of modification and of course, similar patterns of modification are also possible for other oligonucleotides according to the invention. Examples for oligonucleotides, which are derived form oligonucleotides ON173 to ON184, but which have in addition to the replacement of sugar-phosphate backbone units by PNA backbone units, phosphodiester internucleoside modifications a particular positions within the DNA part of the oligonucleotide are:
ON185 (3')-c c a g c c c G G*A G*G-5' (example for SEQ ID NO. 14),
ON186 (3')-c g g a g g c T*T*T G*G-5' (example for SEQ ID NO. 16),
ON187 (3')-g a t g g a G G*T*G G*T-5' (example for SEQ ID NO. 27),
ON188 (3')-g g a g g t G G*T A C*G-5' (example for SEQ ID NO. 28),
ON189 (3')-g g t g g t a C*G G T*T-5' (example for SEQ ID NO. 29),
ON190 (3')-c a c c a g g G T*C*C*G-5' (example for SEQ ID NO. 33),
ON191 (3')-c c a g g g t C*C*G A*C-5' (example for SEQ ID NO. 34),
ON192 (3')-a g g g t c c G A C*G*T-5' (example for SEQ ID NO. 35),
ON193 (3')-g g g t c c G A C*G T*G-5' (example for SEQ ID NO. 36),
ON194 (3')-g g t c c g a C*G T*G G-5' (example for SEQ ID NO. 37),
ON195 (3')-c c g a c g t G G G*T*A-5' (example for SEQ ID NO. 38),
ON196 (3')-c c c c c g a C*G A C*G-5' (example for SEQ ID NO. 56),
wherein
small letters indicate PNA units,
underlined letters indicate hydroxy ethyl glycine-PNA units,
large letters indicate DNA,
" * " is a modified internucleoside bridge, preferably a phosphorothioate bridge.

The above concrete oligonucleotides - particular sequence, particular type(s) of modification(s) at particular positions (specific "pattern of modification") are only examples for different embodiments of the invention. The invention is not limited to these concrete oligonucleotides. Also other combinations of sequence and pattern of modification are possible.

An oligonucleotide according to the invention specifically inhibits the expression of the target protein (which is VEGF) or the target sequence (a nucleic acid which encodes VEGF, preferably VEGF mRNA) respectively. Preferably, an oligonucleotide according to the invention specifically inhibits the expression of VEGF. This results in a reduction in the VEGF protein level in comparison to untreated expression. The specificity can for example be demonstrated by determining the effect of an oligonucleotide according to the invention upon VEGF expression in comparison to the effect of the same oligonucleotide upon beta actin expression, on the mRNA and/or the protein level: upon treatment with an oligonucleotide according to the invention only the VEGF mRNA and/or VEGF protein level were reduced, while e.g. beta actin (a house-keeping protein) mRNA and/or beta-actin protein level remained unchanged. In particular, the effect of an oligonucleotide can be demonstrated by determining the VEGF mRNA and/or the VEGF protein amount (e.g. in comparison to a parallel experiment without the oligonucleotide). For example, the inhibitory effect of the oligonucleotide can be determined in vitro by treating cell cultures with the oligonucleotide. Then, for example the mRNA level can be determined in cell lysate preparations, for example as described in example 4. The VEGF protein level (e.g. absolute amount of VEGF protein in gram or e.g. relative in comparison to an untreated cell in percent) can be determined from the supernatant (e.g. the culture medium) (the amount of secreted VEGF) and/or membrane preparations (the amount of membrane-bound VEGF) and/or cell lysates. The amount of secreted VEGF protein can for example be determined by ELISA, e.g. as described in example 3.

In a particular embodiment of the invention, an oligonucleotide can inhibit the expression of VEGF mRNA and/or reduce the VEGF protein level respectively, e.g. in a cell culture with an IC₅₀ of about 1µM or lower, e.g. 500 nM, 200 nM, 100 nM or less.

Furthermore, the inhibition is specific for an oligonucleotide according to the invention, since only an oligonucleotide which has a particular sequence reduces the VEGF protein and/or VEGF mRNA level. This level is not reduced significantly when an oligonucleotide with a mismatch or a scrambled sequence is used. Such oligonucleotides are used as control oligonucleotides, like oligonucleotides ON200, ON 201, ON203 and ON204. ON200 and ON 201 have two and four mismatches respectively with respect to the sequence of ON16 (SEQ ID NO. 28); but all three oligonucleotides have the same pattern of phosphorothioate modification (positions of " * "). ON203 and ON 204 have two and four mismatches respectively with respect to the sequence of ON24 (SEQ ID NO. 36); but again all three oligonucleotides have the same pattern of phosphorothioate modification (positions of " * "). These four oligonucleotides are used e.g. in comparative experiments with ON16 and ON24 respectively. The control oligonucleotides do not inhibit the expression of VEGF mRNA in cell culture at a concentration of 1 µM and lower (table 3).
ON16 3'-G*G*A G G*T G G*T A C*G-5' antisense oligonucleotide,
ON200 3'-G*G*A G T*G G G*T A C*G-5' 2 mismatches,
ON201 3'-G*G*C G T*G G G*T A A*G-5' 4 mismatches,
ON24 3'-G*G G*T*C*C G A C*G T*G-5' antisense oligonucleotide,
ON203 3'-G*G G*T*C*C A G C*G T*G-5' 2 mismatches, and
ON204 3'-G*G G*C*C*C A G T*G T*G-5' 4 mismatches,
wherein
the position of "mismatches" - with respect to ON16 for ON200 and ON201 and with respect to ON24 for ON203 and ON 204 - are underlined,
ON200 has sequence SEQ ID NO. 89: 3'-GGAGTGGGTACG -5',
ON201 has sequence SEQ ID NO. 90: 3'-GGCGTGGGTA AG -5',
ON203 has sequence SEQ ID NO. 91: 3'-GGGTCCAGCGTG -5',
ON204 has sequence SEQ ID NO. 92: 3'-GGGCCCAGTGTG -5'.

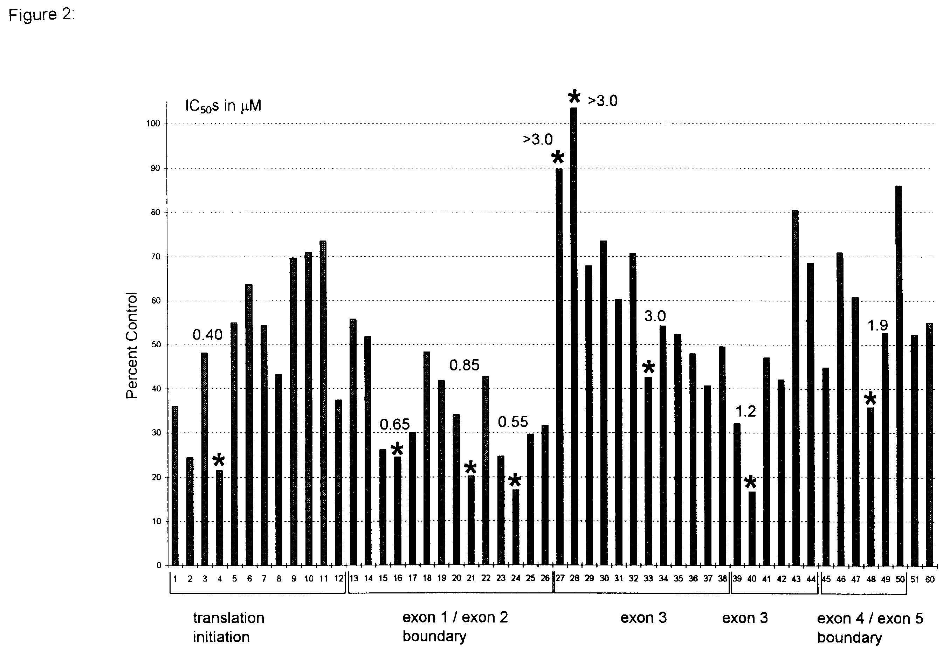

An oligonucleotide according to the invention efficiently inhibits VEGF protein synthesis in cell culture relative to control oligonucleotides. Figure 2 shows the inhibition of VEGF protein secretion by U87 cells treated with one of 52 different 12-mer antisense oligonucleotides at a concentration of 3µM for each oligonucleotide. The corresponding antisense oligonucleotide sequences are summarized in Table 2, which also gives the IC₅₀ values of some oligonucleotides.

An oligonucleotide according to the invention inhibits VEGF protein expression about 55%, preferably about 65% or more, most preferably about 75 % or more relative to control cells, e.g. the amount of secreted VEGF is reduced about 55 %, 65%, 75 % or more when the cell is treated with an oligonucleotide according to the invention at a concentration of 3 µM, preferably even at a at a lower concentration, such as 1 µM or less, preferably 0,5 µM or less (see figure 2).

Preferably an oligonucleotide according to the invention can efficiently inhibit the expression of VEGF (isoforms) in a human cell and/or has the ability to inhibit tumor growth in vertebrates. Preferably, an oligonucleotide according to the invention reduces the VEGF mRNA and/or protein level in tumors of treated individuals relative to untreated individuals. Preferably, an oligonucleotide according to the invention reduces tumor volume in a vertebrate e.g in mice compared to untreated mice or relative to the tumor volume of the same animal determined before treatment.

The invention also relates to a method for the preparation of an oligonucleotide according to the invention. A method for preparation comprises the chemical synthesis of the oligonucleotide. Preferably the chemical synthesis is performed by a standard method known to be used for the synthesis of oligonucleotides, e.g. the phoshoramidite method according to Caruthers (1983) Tetrahedron Letters 24, 245, the H-phosphonate methode (Todd et al. (1957) J. Chem. Soc. 3291 or the phosphotriester methode (Sonveaux (1986) Bioorg. Chem. 14,274; Gait, M.J. "Oilgonucleotide Synthesis, A practical Approach", IRL Press, Oxford,1984) or improved or varied methods derived from these standard methods. An oligonucleotide according to the invention can for example be prepared as described in example 1. Preferably an oligonucleotide according to the invention is synthesized on a solid support by condensing suitably protected monomers (e.g. nucleosides) in order to form internucleoside bridges between these monomers. The invention relates e.g. to a method for preparing an oligonucleotide or a derivative thereof, where a nucleotide unit with a 3'- or a 2'-terminal phosphorus (V) group and a free 5'-hydroxyl or mercapto grouping is reacted with a further nucleotide unit with a phosphorus (III) or a phosphorus (V) grouping in the 3'position, or its activated derivatives and wherein optionally protective groups are used, which can be temporarily introduced in the oligonucleotide in order to protect other functions and which are removed after synthesis, and the oligonucleotide which has been cleaved from the solid support can optionally be converted into a physiologically tolerated salt. In order to synthesize a modified oligonucleotide, standard methods are varied to a certain extent. Those variations are known to a person of skill in the art and are e.g. described in Agrawal S. "Protocols for oligonucleotides and analogs" (1993, Human Press Inc., Totowa, New Jersey). The preparation of modified oligonucleotides is also described in EP 0 710 667, EP 0 680 969, EP 0 464 638, EP 0 593 901, WO 95/01363, EP 0 672 677, EP 0 739 898 and EP 0 552 766. The methods of preparing modified oligonucleotides described in the above documents are hereby incorporated by reference.

The invention further relates to a method of inhibiting the expression of VEGF and/or modulating the expression of a VEGF encoding nucleic acid, wherein an oligonucleotide according to the invention is brought into contact with a VEGF encoding nucleic acid (e.g. mRNA, cDNA) and the oligonucleotide is hybridized to (bind to) this VEGF encoding nucleic acid.

Therefore, the invention also relates to a method, wherein the oligonucleotide is brought into contact with a VEGF encoding nucleic acids (e.g. mRNA; cDNA), for example by introducing the oligonucleotide into a cell by known methods, for example by incubation of cells with said oligonucleotide or a formulation thereof - such formulation may comprise uptake enhancers, such as lipofectin, lipofectamine, cellfectin or polycations (e.g. polylysine).
For example, an oligonucleotide which was incubated previously with cellfectin for e.g. 30 minutes at room temperature is then incubated about 5 hours or less with a cell in order to introduce the oligonucleotide into the cell.

The invention further relates to the use of the oligonucleotide, preferably as antisense oligonucleotide (binding of the oligonucleotide to a VEGF encoding mRNA) or as ribozyme (binding to a VEGF encoding mRNA and cleavage of this mRNA). In another special embodiment of the invention, the oligonucleotide can be used to induce RNAse H cleavage of the VEGF encoding mRNA, thus resulting a reduction in VEGF expression.

The invention relates to the use of the oligonucleotide for modulating and also totally or partially inhibiting the expression of VEGF (e.g. VEGF₁₂₁, VEGF₁₆₅, VEGF₁₈₉, VEGF₂₀₆) and/or splice variants thereof and/or mutants thereof, for example for totally or partially inhibiting translation of VEGF encoding mRNA.

The invention relates to the use of an oligonucleotide for inhibiting, preventing or modulating angiogenesis, neovascularisation, tumor growth and metastasis, in particular in vertebrate. The invention in general relates to the use of an oligonucleotide according to the invention for the treatment or the prevention of diseases, in which VEGF is overexpressed. Such diseases in which VEGF is over expressed are for example cancer, age-related macular degeneration, diabetic retinopathy, psoriasis, rheumatoide arthritis and other inflammatory diseases.

The invention furthermore relates to the use of the oligonucleotide as pharmaceutical and to the use of the oligonucleotide for preparing a pharmaceutical composition. In particular, the oligonucleotide can be used in a pharmaceutical composition, which is employed for preventing and/or treating diseases which are associated with the expression or an overexpression (increased expression) of VEGF and for treating of diseases in which VEGF or its overexpression is the causative factor or is involved.

The invention furthermore relates to a pharmaceutical composition which comprise an oligonucleotide and/or its physiologically tolerated salts in addition to pharmaceutically unobjectable excipients or auxiliary substances.

The invention relates to a pharmaceutical composition which comprises at least one oligonucleotide according to the invention that can be used for the treatment of diseases which are associated with abnormal vascular permeability, cell proliferation, cell permeation, angiogenesis, neovascularization, tumor cell growth and the metastasis of neoplastic cells.

The invention further relates to a method for preparing a pharmaceutical composition, which comprises mixing of one or more oligonucleotides according to the invention with physiologically acceptable exipient and optionally additional substances, e.g. if appropiate with suitable additives and/or auxiliaries.

The invention relates in particular to the use of an oligonucleotide or a pharmaceutical composition prepared thereof for the treatment of cancer, e.g. for inhibiting tumor growth and tumor metastasis, and for the treatment of diabetic retinopathy, age-related macular degeneration, psoriasis, rheumatoid arthritis and other inflammatory diseases. For example the oligonucleotide or a pharmaceutical composition prepared thereof may be used for the treatment of solid tumors, like breast cancer, lung cancer, head and neck cancer, brain cancer, abdominal cancer, colon cancer, colorectal cancer, Esophagus cancer, gastrointestinal cancer, Glioma, liver cancer, tongue cancer, neuroblastoma, osteosarcoma, ovarian cancer, pancreatic cancer, prostata cancer, Retinoblastoma, Wilm's tumor, multiple myeloma and for the treatment of skin cancer, like melanoma, for the treatment of lymphomas and blood cancer. The invention further relates to the use of an oligonucleotide according to the invention or a pharmaceutical composition prepared thereof for inhibiting VEGF expression and/or for inhibiting accumulation of ascites fluid and pleural effusion in different types of cancer e.g. breast cancer, lung cancer, head cancer, neck cancer, brain cancer, abdominal cancer, colon cancer, colorectal cancer, Esophagus cancer, gastrointestinal cancer, Glioma, liver cancer, tongue cancer, neuroblastoma, osteosarcoma, ovarian cancer, pancreatic cancer, prostata cancer, Retinoblastoma, Wilm's tumor, multiple myeloma, skin cancer, melanoma, lymphomas and blood cancer. Due to the inhibitory effect on VEGF expression and/or ascites fluid and pleural effusion, an oligonucleotide according to the invention or a pharmaceutical composition prepared thereof can enhance the quality of live. In a preferred embodiment of the invention, the oligonucleotide or a pharmaceutical composition thereof can inhibits accumulation of ascites fluids in ovarian cancer.

The invention furthermore relates to the use of an oligonucleotide or a pharmaceutical composition thereof, e.g. for treating cancer or for preventing tumor metastasis, or for treating age-related macular degeneration, rheumatoid arthritis, psoriasis and diabetic retinopathy in combination with other pharmaceuticals and/or other therapeutic methods, e.g. with known pharmaceuticals and/or known therapeutic methods, such as for example those, which are currently employed for treating cancer and/or for preventing tumor metastasis. Preference is given to a combination with radiation therapy and chemotherapeutic agents, such as cis-platin, cyclophosphamide, 5-fluorouracil, adriamycin, daunorubicin or tamoxifen.

The oligonucleotide and/or its physiologically tolerated salt can be administered to an animal, preferably a mammalian, and in particular a human, on its own, in mixture with another oligonucleotide (or its physiologically tolerated salt), or in the form of a pharmaceutical composition which permit topical, percutaneous, parenteral or enteral use and which comprise, as the active constituent, an effective dose of at least one oligonucleotide in addition to customary pharmaceutically unobjectable excipients and auxiliary substances. Such pharmaceutical composition normally comprises from about 0.1 to 90% by weight of the therapeutically active oligonucleotide(s). The dose can vary within wide limits and is to be adjusted to the individual circumstances in each individual case. In order to treat psoriasis, preference is given to a topical use. In the case of cancer, preference is given to infusions, oral and rectal administration, or nasal application in an aerosol, preferable in the case of lung cancer, while in the case of diabetic retinopathy, preference is given to a topical, intravitreal and oral administration.

A pharmaceutical composition might be prepared in a manner known per se (e.g. Remingtons Pharmaceutical Sciences, Mack Publ. Co., Easton, PA (1985)), with pharmaceutically inert inorganic and/or organic excipients being used. Lactose, corn starch and/or derivatives thereof, talc, stearic acid and/or its salts, etc. can, for example, be used for preparing pills, tablets, coated tablets and hard gelatin capsules. Examples of excipients for soft gelatin capsules and/or suppositories are fats, waxes, semisolid and liquid polyols, natural and/or hardened oils, etc. Examples of suitable excipients for preparing solutions and/or syrups are water, sucrose, invert sugar, glucose, polyols, etc. Suitable excipients for preparing injection solutions are water, alcohols, glycerol, polyols, vegetable oils, etc. Suitable excipients for microcapsules, implants and/or rods are mixed polymers of glycolic acid and lactic acid. In addition, liposome formulations which are e.g. described in N. Weiner, (Drug Develop Ind Pharm 15 (1989) 1523), "Liposome Dermatics" (Springer Verlag 1992) and Hayashi (Gene Therapy 3 (1996) 878). The pharmaceutical composition may also comprise formulation, which enhances the oral availability of the oligonucleotide, such as enhancers of intestinal permeabilization, e.g. mannitol, urea, bile salts, such as CDCA (chenodexoycholate) (2 %).
Dermal administration can also be effected, for example, using ionophoretic methods and/or by means of electroporation. Furthermore, use can be made of lipofectins and other carrier systems, for example those which are used in gene therapy. Systems which can be used to introduce oligonucleotides in a highly efficient manner into eukaryotic cells or into the nuclei of eukaryotic cells are particularly suitable. A pharmaceutical composition may also comprise two or more different oligonucleotides and/or their physiologically tolerated salts and, furthermore, in addition to at least one oligonucleotide, one or more different therapeutically active ingredients.

In addition to the active ingredients and excipients, a pharmaceutical composition can also comprise additives, such as fillers, extenders, disintegrants, binders, lubricants, welling agents, stabilizing agents, emulsifiers, preservatives, sweeteners, dyes, flavorings or aromatizing agents, thickeners, diluents or buffering substances, and, in addition, solvents and/or solubilizing agents and/or agents for achieving a slow release effect, and also salts for altering the osmotic pressure, coating agents and/or antioxidants.
Figure 1: Figure 1 (part A to E) shows the localization of tested VEGF antisense oligonucleotides (SEQ ID NO. 13 to SEQ ID NO. 72) with respect to the cDNA sequence of the VEGF clone (both strands), for which the nucleotide sequence is given in table 1. Also the localization of the core regions 1 to 6 and of sequences SEQ ID NO. 1 to SEQ ID NO. 12 are shown (underlined parts of the sequence).
Figure 2: Summarizes the inhibitory effects of different oligonucleotides (each of them used at a concentration of 3 µM) on VEGF protein secretion in cell culture (secretion by human U87-MG cells). The inhibitory effects where shown relative to control cell that were not treated with the oligonucleotides (amount of secreted VEGF from cells treated with the oligonucleotides to amount of secreted VEGF from cells not treated with the oligonucleotides). Oligonucleotides: The results show, that ON2, ON4, ON15, ON16, ON17, ON21, ON22, ON23, ON24, ON25, ON26 ON39 and ON40 show the best inhibitory effect relative to control cells. Abbreviations: 1 is ON 300 2 is ON 2, 3 is ON 301, 4 is ON 4, 5 is ON 302, 6 is ON 303, 7 is ON 304, 8 is ON 305, 9 is ON 306, 10 is ON 307, 11 is ON 308, 12 is ON 309, 13 is ON310, 14 is ON 311, 15 is ON 15, 16 is ON16, 17 is ON17, 18 is ON 312, 19 is ON 313, 20 is ON314, 21 is ON 33, 22 is ON22, 23 is ON 23, 24 is ON 24, 25 is ON 25, 26 is ON 26, 27 is ON 315, 28 is ON 316, 29 is ON 317, 30 is ON 318, 31 is ON 319, 32 is ON 320, 33 is ON 321, 34 is ON 322, 35 is ON323, 36 is ON324, 37 is ON 325, 38 is ON 326, 39 is ON 39, 40 is ON 40, 41 is ON 327, 42 is ON 328, 43 is ON 329, 44 is ON 330, 45 is ON 331, 46 is ON 332, 47 is ON 333, 48 is ON 334, 49 is ON 335, 50 is ON 336, 51 is ON 337 and 60 ON 345.

### Examples:

### Example 1: Oligonucleotide synthesis

Oligonucleotides (ON s) were synthesized using an Applied Biosystems 394 DNA synthesizer (Perkin Elmer Applied Biosystems, Inc., Foster City, USA) and standard phosphoramidite chemistry. After coupling, phosphorothioate linkages were introduced by sulfurization using the Beaucage reagent followed by capping with acetic anhydride an*d N*-methylimidazole. After cleavage from the solid support and final deprotection by treatment with concentrated ammonia, ON s were purified by polyacrylamide gel electrophoresis. The 2'-*O-methyl* modified ON s were prepared by replacing the standard phosphoramidites in the corresponding cycle with 2'-O-methyl ribonucleoside phophoramidites. All ON s were analysed by negative ion electrospray mass spectroscopy (Fisons Bio-Q) which in all cases confirmed the calculated mass. The C16-modified oligonucleotides were synthesised using hexadecyloxy (cyanoethoxy) N,N-diisopropyl aminophosphane as phosphitylating reagent in the last step of oligonucleotide synthesis in place of a standard amidite, or by starting from a correspondingly derivatized solid support. The triethylene glycol linker is commercially available from Glen Research Corporation. The 2'-phosphoramidite of adenosin or cordycepin were obtained from Chem. Genes Corporation and Chemogen Corporation, respectively. The introduction of 5'-phosphates or thiophosphate residues was carried out as described previously (Uhlmann and Engels (1986) Tetrahedron Lett. 27, 1023). The PNA-DNA chimeras are prepared as described in EP 0 672 677.

Analysis of the oligonucleotides was done by
a) Analytical gel electrophoresis in 20% acrylamide, 8M urea, 45µM tris-borate buffer, pH 7.0 and/or
b) HPLC-analysis: Waters GenPak FAXcolumn, gradient CH₃CN (400ml), H₂O (1.61), NaH₂PO₄ (3.1g), NaCl (11.7g), pH6.8 (0.1M an NaCl) after CH₃CN (400ml), H₂O (1.61), NaH₂PO₄ (3.1g), NaCl (175.3g), pH6.8 (1.5M an NaCl) and/or
c) capillary electrophoresis using a Beckmann capillary eCAP™, U100P Gel Column, 65 cm length, 100 mm I.D., window 15 cm from one end, buffer 140 µM Tris, 360mM borate, 7M urea and/or
d) negative ion electrospray mass spectrometry which in all cases confirmed the expected mass values.

The methods for analyzing oligonucleotides according to a), b), c) and d) are known to a person of skill in the art. These methods are for example described in Schweitzer and Engel "Analysis of oligonucleotides" (in "Antisense - from technology to therapy", a laboratrory manual and textbook, Schlingensiepen et al. eds., Biol. Science Vol. 6 (1997) p. 78- 103).

The following oligonucleotides were prepared (see description): and tested:
ON 300 3'-G*G*C*C A G C*C*C G G*A-5' (Sequence SEQ ID NO. 13),
ON 2 3'-C*C*A G C*C*C*G G A G*G-5' (Sequence SEQ ID NO. 14),
ON 301 3'-G*C*C*C G G A G G*C*T*T-5' (Sequence SEQ ID NO. 15),
ON 4 3'-C*G*G A G G C*T*T*T G*G-5' (Sequence SEQ ID NO. 16),
ON 302 3'-G*G*C T*T*T G G T*A C*T-5' (Sequence SEQ ID NO. 17),
ON 303 3'-T*T*G G*T A C*T*T G A*A-5' (Sequence SEQ ID NO. 18),
ON 304 3'-G*G*T A C*T*T*G A A A*G-5' (Sequence SEQ ID NO. 19),
ON 305 3'-C*T*T*G A A A G A*C*G*A-5' (Sequence SEQ ID NO. 20),
ON 306 3'-G*A*A A G A*C*G A*C A*G-5' (Sequence SEQ ID NO. 21),
ON 307 3'-G*A*C*G A C*A G A A*C*C-5' (Sequence SEQ ID NO. 22),
ON 308 3'-G*A*C*A G A A C*C*C A*C-5' (Sequence SEQ ID NO. 23),
ON 309 3'-G*A A C*C*C A*C G*T A*A-5' (Sequence SEQ ID NO. 24),
ON 310 3'-C*G*A C*G A G A*T G G*A-5' (Sequence SEQ ID NO. 25),
ON 311 3'-C*G*A G A*T*G G A G G*T-5' (Sequence SEQ ID NO. 26),
ON 15 3'-G*A*T G G A G G*T*G G*T-5' (Sequence SEQ ID NO. 27),
ON 16 3'-G*G*A G G*T G G*T A C*G-5' (Sequence SEQ ID NO. 28),
ON 17 3'-G*G*T*G G T*A C*G G T*T-5' (Sequence SEQ ID NO. 29),
ON 312 3'-G*G*T A*C G G T*T*C A*C-5' (Sequence SEQ ID NO. 30),
ON 313 3'-A*C*G G T*T*C A C*C A*G-5' (Sequence SEQ ID NO. 31),
ON 314 3'-G*G*T T*C*A C*C A G G*G-5' (Sequence SEQ ID NO. 32),
ON 21 3'-C*A*C*C A G G G T*C*C*G-5' (Sequence SEQ ID NO. 33),
ON 22 3'-C*C*A G G G T*C*C G A*C-5' (Sequence SEQ ID NO. 34),
ON 23 3'-A*G*G G T*C*C G A C*G*T-5' (Sequence SEQ ID NO. 35),
ON 24 3'-G*G G*T*C*C G A C*G T*G-5' (Sequence SEQ ID NO. 36),
ON 25 3'-G*G*T C*C*G A C*G T*G G-5' (Sequence SEQ ID NO. 37),
ON 26 3'-C*C*G A*C G*T G G G*T*A-5' (Sequence SEQ ID NO. 38),
ON 315 3'-C*C*A C*T*T*C A A G T*A-5' (Sequence SEQ ID NO. 39),
ON 316 3'-C*T*T*C A A G*T A C*C*T-5' (Sequence SEQ ID NO. 40),
ON 317 3'-C*A*A G*T A C*C*T A C*A-5' (Sequence SEQ ID NO. 41),
ON 318 3'-G*T*A C*C*T A C*A G A*T-5' (Sequence SEQ ID NO. 42),
ON 319 3'-A*C*C*T A*C A G A*T A*G-5' (Sequence SEQ ID NO. 43),
ON 320 3'-C*T*A*C A G A*T A G*T*C-5' (Sequence SEQ ID NO. 44),
ON 321 3'-C*A*G A*T A G*T*C G C*G-5' (Sequence SEQ ID NO. 45),
ON 322 3'-G*A*T A G T*C G*C*G T*C-5' (Sequence SEQ ID NO. 46),
ON 323 3'-G*T*C G*C G*T*C G A T*G-5' (Sequence SEQ ID NO. 47),
ON 324 3'-C*G*C*G T*C G A*T G A*C-5' (Sequence SEQ ID NO. 48),
ON 325 3'-C*G*T*C G A*T G A*C G*G-5' (Sequence SEQ ID NO. 49),
ON 326 3'-C*G*A*T G A*C G G*T A*G-5' (Sequence SEQ ID NO. 50),
ON 39 3'-A*C*G C*C*C C*C G A C*G-5' (Sequence SEQ ID NO. 55),
ON 40 3'-C*C*C*C C*G A*C G A C*G-5' (Sequence SEQ ID NO. 56),
ON 327 3'-C*G*A C G T*T A*C*T G*C-5' (Sequence SEQ ID NO. 57),
ON 328 3'-C*T*C C*C G G A C*C*T*C-5' (Sequence SEQ ID NO. 58),
ON 329 3'-C*G*G A C*C*T*C A C A*C-5' (Sequence SEQ ID NO. 59),
ON 330 3'-G*A*C*C T*C A*C A C A*C-5' (Sequence SEQ ID NO. 60),
ON 331 3'-G*A*T G T*C G T*G T*T*G-5' (Sequence SEQ ID NO. 67),
ON 332 3'-C*G*T G T*T G T*T T*A*C-5' (Sequence SEQ ID NO. 68),
ON 333 3'-C*A*C*T T A*C G T*C T*G-5' (Sequence SEQ ID NO. 69),
ON 334 3'-C*T*T A*C G T*C*T G G*T-5' (Sequence SEQ ID NO. 70),
ON 335 3'-C*G*T C*T G G T*T T*C*T-5' (Sequence SEQ ID NO. 71),
ON 336 3'-C*T*G G T*T T*C T*T T*C-5' (Sequence SEQ ID NO. 72),
ON 337 3'-G*T*G G*T A*C G T*C*T*A-5' (Sequence SEQ ID NO. 61),
ON 338 3'-G*G*T A*C G T*C*T A A*T-5' (Sequence SEQ ID NO. 62),
ON 339 3'-C*G*T*C T*A A T*A C*G*C-5' (Sequence SEQ ID NO. 63),
ON 340 3'-C*T*A A*T A*C G C*C*T*A-5' (Sequence SEQ ID NO. 64),
ON 341 3'-C*G*C C*T A G T*T*T G*G-5' (Sequence SEQ ID NO. 65),
ON 342 3'-A*G*T*T*T G G A G*T G*G-5' (Sequence SEQ ID NO. 66),
ON 343 3'-G*C*T*C A T*G T*A G A*A-5' (Sequence SEQ ID NO. 51),
ON 58 3'-G*T*A G A A G*T T*C*G*G-5' (Sequence SEQ ID NO. 52),
ON 344 3'-G*A*A G T*T*C*G G*T A*G-5' (Sequence SEQ ID NO. 53),
ON 345 3'-C*G*G*T A G G A*C A*C*A-5' (Sequence SEQ ID NO. 54),
ON104 3'-G*G*G T*C*C G A C*G T*G-5',
ON105 3'-G*G*G T*C*C G A C*G*T*G-5',
ON106 3'-G*G*G T*C*C G A C*G*T G-5',
ON114 3'-C*C*A G C*C*C*G G A G*G-5',
ON115 3'-C*G*G A G G C*T*T*T G*G-5',
ON116 3'-G*A*T G G A G G*T*G G*T-5',
ON117 3'-G*G*A G G*T G G*T A C*G-5',
ON118 3'-G*G*T*G G T*A C*G G T*T-5',
ON119 3'-C*A*C*C A G G G T*C*C*G-5',
ON120 3'-C*C*A G G G T*C*C G A*C-5',
ON121 3'-A*G*G G T*C*C G A C*G*T-5',
ON122 3'-G*G G*T*C*C G A C*G T*G-5',
ON123 3'-G*G*T C*C*G A C*G T*G G-5',
ON124 3'-C*C*G A*C G*T G G G*T*A-5',
ON125 3'-C*C*C*C C*G A*C G A C*G-5',
ON139 3'-C*C*A G C*C*C*G G A G*G-5',
ON140 3'-C*G*G A G G C*T*T*T G*G-5',
ON141 3'-G*A*T G G A G G*T*G G*T-5',
ON142 3'-G*G*A G G*T G G*T A C*G-5',
ON143 3'-G*G*T*G G T*A C*G G T*T-5',
ON144 3'-C*A*C*C A G G G T*C*C*G-5',
ON145 3'-C*C*A G G G T*C*C G A*C-5',
ON146 3'-A*G*G G T*C*C G A C*G*T-5',
ON147 3'-G*G G*T*C*C G A C*G T*G-5,
ON148 3'-G*G*T C*C*G A C*G T*G G-5',
ON149 3'-C*C*G A*C G*T G G G*T*A-5',
ON150 3'-A*C*G C*C*C C*C G A C*G-5',
ON151 3'-C*C*C*C C*G A*C G A C*G-5',
ON152 3'-G*T*A G A A G*T T*C*G*G-5',
ON153 3'-C*C*A G C*C*C*G G A G*G-C16-5',
ON154 3'-C*G*G A G G C*T*T*T G*G-C16-5',
ON155 3'-G*A*T G G A G G*T*G G*T-C16-5',
ON156 3'-G*G*A G G*T G G*T A C*G-C16-5',
ON157 3'-G*G*T*G G T*A C*G G T*T-C16-5',
ON158 3'-C*A*C*C A G G G T*C*C*G-C16-5',
ON159 3'-C*C*A G G G T*C*C G A*C-C16-5',
ON160 3'-A*G*G G T*C*C G A C*G*T-C16-5',
ON161 3'-G*G G*T*C*C G A C*G T*G-C16-5',
ON162 3'-G*G*T C*C*G A C*G T*G G-C16-5',
ON163 3'-C*C*G A*C G*T G G G*T*A-C16-5',
ON164 3'-C*C*C*C C*G A*C G A C*G-C16-5',
ON165 3'-teg-G*G*G T*C*C G A C*G T*G -5',
ON166 3'-teg-G*G*G T*C*C G A C*G T*G -5',
ON167 3'-teg-G*G*G T*C*C G A C*G T*G -5',
ON346 3'-C*C*A G C*C*C*G G A G*G-vitE -5',
ON347 3'-G*A*T G G A G G*T*G G*T-vitE -5',
ON348 3'-G*G*A G G*T G G*T A C*G-vitE -5',
ON349 3'-G*G*T*G G T*A C*G G T*T-vitE -5',
ON350 3'-C*A*C*C A G G G T*C*C*G-vitE -5',
ON351 3'-C*C*A G G G T*C*C G A*C-vitE -5',
wherein
"*" is a phosphorothioate internucleoside bridge,
a underlined "N " is a 2'-O-methylribonucleoside (in this case "T" is 2'-O-mehtyluridine),
"teg" is a triethyleneglycol phophate linker,
"C16" is a hexadecylphosphate, and
"vitE" is a vitamine E glycerol phosphate.

### Example 2: Treatment of cells with antisense oligonucleotides

The cells are plated in 96-well plates at 30,000 cells/well, 150 µl medium per well (medium depends on cell type). The next day, Cellfectin (Gibco-BRL) is diluted to 400 ug/ml in water (solution A). Oligonucleotides are diluted to 40X the final desired concentration in water (solution B). Equal amounts 21 of solutions A and B are mixed, to give the desired volume of a solution that is 200 ug/ml Cellfectin and 20X oligonucleotide, and the mixture left at room temperature for 30 minutes. After 30 minutes, 19 volumes of Optimem (Gibco-BRL) is added to give a final solution that is 10 ug/ml Cellfectin and 1X oligonucleotide (solution C). Medium is removed from the cells, the wells are washed 2X with Optimem, and 150 µl solution C added to each well. The plates are then returned to the incubator. After 5 hours, the Cellfectin/oligonucleotide solution is removed and replaced with 150 µl of regular growth medium. VEGF protein and mRNA assays are performed beginning 19 hours later.

### Example 3: Inhibition of VEGF expression by antisense oligonucleotides in cell culture (VEGF protein assay).

Samples of conditioned medium are taken from the desired wells and assayed for the presence of human VEGF using the human VEGF ELISA kit from R & D systems. The assay protocol is the one provided by the supplier with the kit. The inhibition of VEGF expression in U87-MG cells by different 12-mer antisense oligonucleotides is shown in Table 2 and figure 2. There are several antisense oligonucleotides, modified as partial phosphorothioates, which inhibit the VEGF expression at 3 µM oligonucleotide concentration by about 80% (e.g. ON 2, ON 4, ON 15, ON 16, ON 17, ON 24, ON 40) while other oligonucleotides are virtually inactive under the same conditions (e.g. ON 315 and ON 316). The phosphorothioate pattern in the 12-mers can be varied within the limits of partially modified oligonucleotides as outlined in the description. Thus, ON 24, ON 104, ON 105 and ON 106 show about the same inhibitory effect, although ON 104 proved to be somewhat more active than the other three oligonucleotides of the same sequence. Partial derivatization as 2'-O-methyl RNA, e.g as in ON 117, further enhances the inhibitory activity as compared to the DNA compound ON 16 of the same sequence.

### Example 4: VEGF mRNA assay

Medium is removed from the 96 well plates described above, and cell lysates are prepared from the remaining cells for quantitation of VEGF mRNA by the Applied Biosystems 7700 Analyser. For determining the mRNA levels, the data are normalized to the amount of β-actin levels detected in the same samples.

### Example 5; Determination of IC(50)- values

The IC50s are calculated based on a value of 100% for the amount of VEGF protein or mRNA in cells treated with Cellfectin but no oligonucleotide. For the ELISA, the amount of VEGF in the conditioned medium is normalized to the cell number in each sample. The cell number is determined by using the CYQuant assay (Molecular Probes, Inc.).

### Example 6: In vivo studies

In vivo experiments can e.g. be performed with 4 - 6 week old female nude (nu/nu) mice, in which tumors can previously be grown by subcutaneous implantation of cells (e.g. 2,000,000 cells in 200 µl for U87-MG). Oligonucleotides can be dissolved in phosphate buffered saline and be injected subcutaneously or intravenously (tailvein) in a volume of 100 µl. 2 x 10⁶ U87-MG. For example, when tumor cells were implanted s.c. on day 0, drug treatment can start on day 1 to 4 by administering the oligonucleotide by daily i.v. tailvein injection.

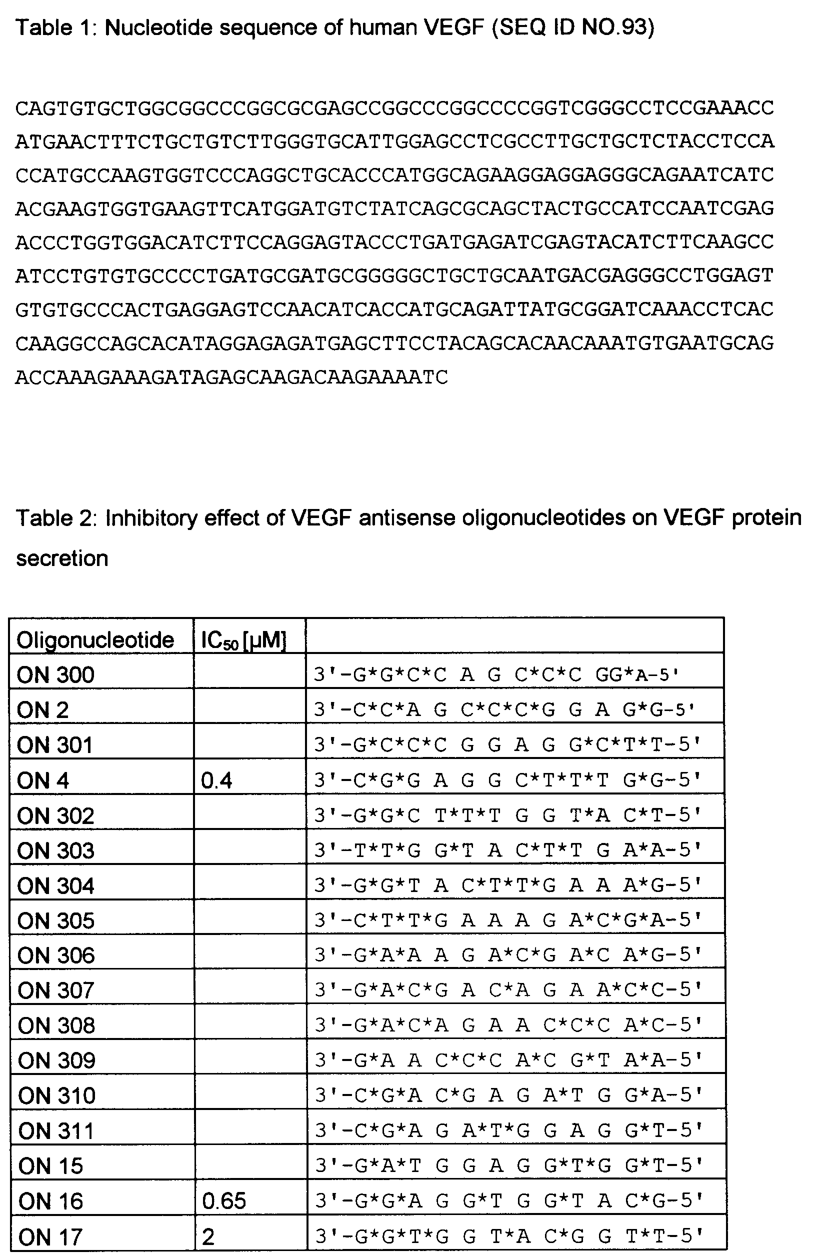

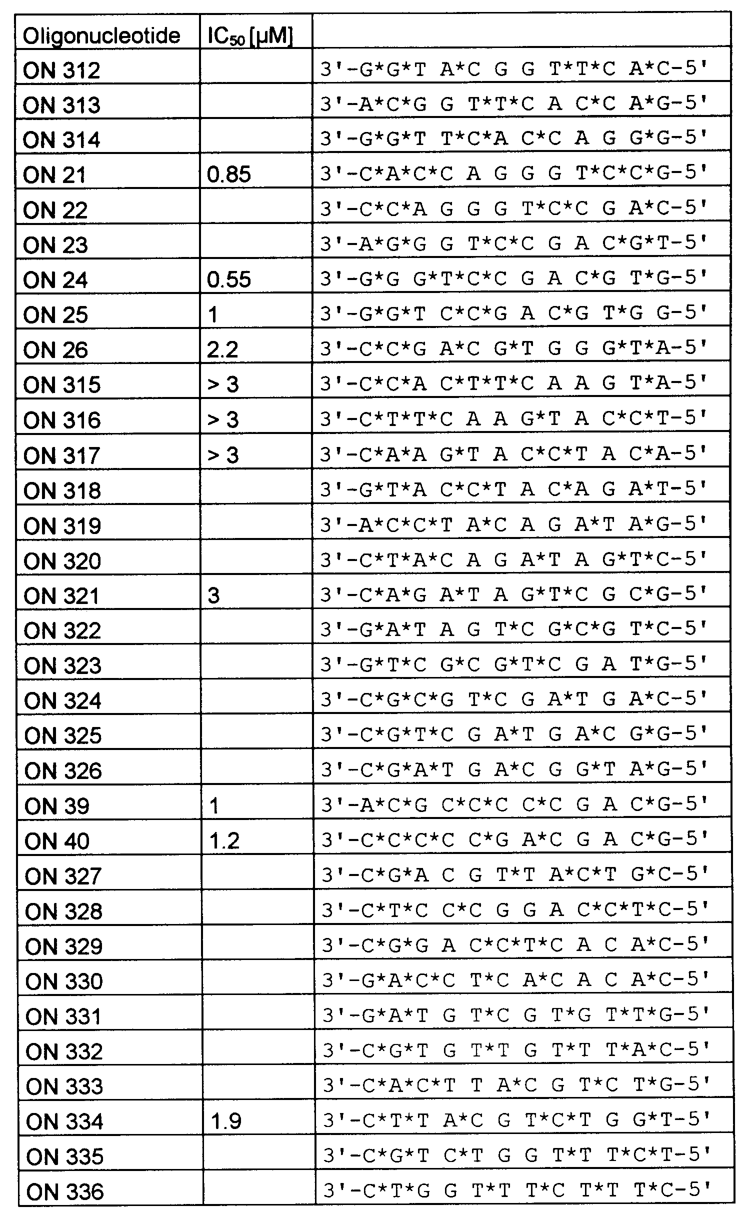

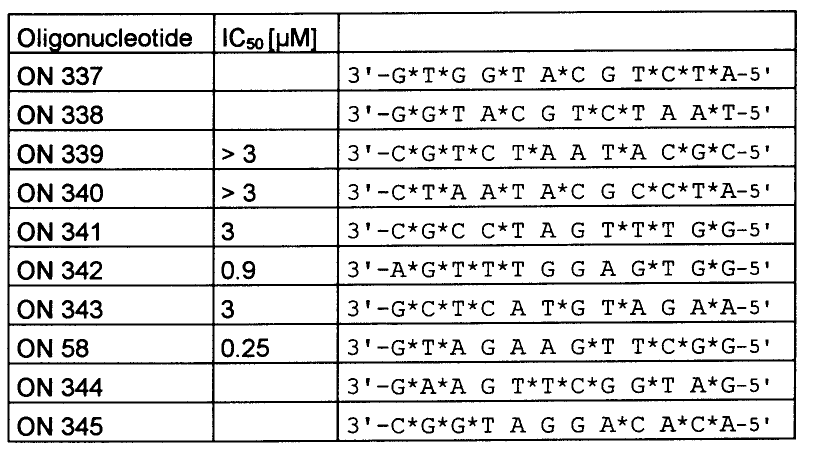

**Table 3**

| Inhibitory effect of oligonucleotides ON18 and ON24 in comparison to oligonucleotides which have 2 and 4 mismatches within the sequence respectively in comparison to oligonucleotides ON18 and ON 24 respectively. | | |
|---|---|---|
| Oligonucleotide | IC₅₀ [µM] | Remarks |
| ON18 | 0.65 | antisense |
| ON200 | not active | 2 mismatches |
| ON201 | not active | 4 mismatches |
| | | |
| ON24 | 0.55 | antisense |
| ON203 | not active | 2 mismatches |
| ON204 | not active | 4 mismatches |

## Claims

1. A short oligonucleotide or a derivative thereof, which has a length of 10 to 15 nucleotides and which corresponds to a part of a VEGF encoding sequence, wherein the part of the VEGF encoding sequence to which the oligonucleotide corresponds to has one of the sequences SEQ ID NO. 1, SEQ ID NO. 2, SEQ ID NO. 3, SEQ ID NO. 4, SEQ ID NO. 5 or SEQ ID NO. 6 or a part thereof,
wherein
SEQ ID NO. 1 is 5'- CCCGGCCCCGGTCGGGCCTCCG - 3',
SEQ ID NO. 2 is 5'- CGGGCCTCCGAAACC -3',
SEQ ID NO. 3 is 5'- GCTCTACCTCCACCATGCCAA -3',
SEQ ID NO. 4 is 5'- GTGGTCCCAGGCTGCACCCATGGC -3',
SEQ ID NO. 5 is 5'- CATCTTCAAGCCATCC -3', and
SEQ ID NO. 6 is 5'- TGCGGGGGCTGCTGC -3'.

2. An oligonucleotide as claimed in claim 1, which has one of the sequences SEQ ID NO. 7 to SEQ ID NO. 12 or a part thereof,
wherein
SEQ ID NO. 7 is 3'- GGGCCGGGGCCAGCCCGGAGGC-5'
SEQ ID NO. 8 is 3'- GCCCGGAGGCTTTGG -5',
SEQ ID NO. 9 is 3'- CGAGATGGAGGTGGTACGGTT -5',
SEQ ID NO. 10 is 3'- CACCAGGGTCCGACGTGGGTACCG -5',
SEQ ID NO. 11 is 3'- GTAGAAGTTCGGTAGG -5', and
SEQ ID NO. 12 is 3'- ACGCCCCCGACGACG -5'

3. An oligonucleotide as claimed in one or more of claims 1 or 2, wherein the oligonucleotide has a length of 12 nucleotides.

4. An oligonucleotide as claimed in one or more of claims 1 to 3, wherein the oligonucleotide has a one of the sequences SEQ ID NO. 14, SEQ ID NO. 16, SEQ ID NO. 27, SEQ ID NO. 28, SEQ ID NO. 29, SEQ ID NO. 33, SEQ ID NO. 34, SEQ ID NO. 35, SEQ ID NO. 36, SEQ ID NO. 37, SEQ ID NO. 38, SEQ ID NO. 52, SEQ ID NO. 55 or SEQ ID NO. 56,
wherein
SEQ ID NO. 14 is 3'- CCAGCCCGGAGG -5',
SEQ ID NO. 16 is 3'- CGGAGGCTTTGG -5',
SEQ ID NO. 27 is 3'- GATGGAGGTGGT -5',
SEQ ID NO. 28 is 3'- GGAGGTGGTACG -5',
SEQ ID NO. 29 is 3'- GGTGGTACGGTT -5',
SEQ ID NO. 33 is 3'- CACCAGGGTCCG -5',
SEQ ID NO. 34 is 3'- CCAGGGTCCGAC -5',
SEQ ID NO. 35 is 3'- AGGGTCCGACGT -5',
SEQ ID NO. 36 is 3'- GGGTCCGACGTG -5',
SEQ ID NO. 37 is 3'- GGTCCGACGTGG -5',
SEQ ID NO. 38 is 3'- CCGACGTGGGTA -5',
SEQ ID NO. 52 is 3'- GTAGAAGTTCGG -5',
SEQ ID NO. 55 is 3'- ACGCCCCCGACG -5', and
SEQ ID NO. 56 is 3'- CCCCCGACGACG -5'.

5. An oligonucleotide as claimed in one or more of claims 1 to 4, wherein the oligonucleotide has one or more modifications and wherein each modification is located at a particular phosphodiester internucleoside bridge and/or a particular β-D-2'-deoxyribose unit and/or a particular natural nucleoside base position in comparison to an oligonucleotide of the same sequence which is composed of natural DNA.

6. An oligonucleotide as claimed in one or more of claims 1 to 5, wherein each modification is independently selected from
a) the replacement of a phosphodiester internucleoside bridge located at the 3'-and/or the 5'- end of a nucleoside by a modified internucleoside bridge,
b) the replacement of phosphodiester bridge located at the 3'- and/or the 5'-end of a nucleoside by a dephospho bridge,
c) the replacement of a sugar phosphate unit from the sugar phosphate backbone by another unit,
d) the replacement of a β-D-2'-deoxyribose unit by a modified sugar unit,
e) the replacement of a natural nucleoside base by a modified nucleoside base,
f) the conjugation to a molecule which influences the properties of the oligonucleotide,
g) the conjugation to a 2'5'-linked oligoadenylate or a derivative thereof, optionally via an appropriate linker, and
h) the introduction of a 3'-3' and/or a 5'-5' inversion at the 3' and/or the 5' end of the oligonucleotide.

7. An oligonucleotide as claimed in one or more of claims 1 to 6, wherein each modification is independently selected from
a) the replacement of a phosphodiester internucleoside bridge located at the 3'-and/or the 5'- end of a nucleoside by a modified internucleoside bridge, wherein the modified internucleoside bridge is selected from phosphorothioate, phosphorodithioate, NR¹R^{1'}-phosphoramidate, boranophosphate, phosphate-(C₁-C₂₁)-O-alkyl ester, phosphate-[(C₆-C₁₂)aryl-((C₁-C₂₁) -O-alkyl]ester, (C₇-C₁₂)-α-hydroxmethylaryl, (C₁-C₈)alkyl-phosphonate and/or (C₆-C₁₂)-arylphosphonate bridges,
wherein R¹ and R^{1'} are, independently of each other, hydrogen, (C₁-C₁₈)-alkyl, (C₆-C₂₀)-aryl, (C₆-C₁₄)-aryl-(C₁-C₈)-alkyl, preferably hydrogen, (C₁-C₈)-alkyl and/or methoxyethyl, or
R¹ and R^{1'} form, together with the nitrogen atom carrying them, a 5-6-membered heterocyclic ring which can additionally contain a further heteroatom from the group O, S and N;
b) the replacement of phosphodiester bridge located at the 3'- and/or the 5'-end of a nucleoside by a dephospho, wherein the dephospho bridge is selected from the dephospho bridges formacetal, 3'-thioformacetal, methylhydroxylamine, oxime, methylenedimethyl-hydrazo, dimethylenesulfone and silyl groups;
c) the replacement of a sugar phosphate unit from the sugar phosphate backbone by another unit, wherein the other unit is selected from morpholino-derivative units, polyamide nucleic acid backbone units, and phosphonic acid monoester nucleic acid backbone units;
d) the replacement of a β-D-2'-deoxyribose unit by a modified sugar unit, wherein the modified sugar unit is selected from β-D-ribose, α-D-2'-deoxyribose, L-2'-deoxyribose, 2'-F-2'-deoxyribose, 2'-O-(C₁-C₆)alkyl-ribose, 2'-O-(C₂-C₆)alkenylribose, 2'-[O-(C₁-C₆)alkyl-O-(C₁-C₆)alkyl]-ribose, 2'-NH₂-2'-deoxyribose, β-D-xylofuranose, α-arabinofuranose, 2,4-dideoxy-β-D-erythro-hexo-pyranose, carbocyclic and/or open-chain sugar analogs and/or bicyclosugar analogs;
e) the replacement of a natural nucleoside base by a modified nucleoside base, wherein the modified nucleoside base is selected from uracil, hypoxanthine, 5-(hydroxymethyl)uracil, N²-Dimethylguanosine, 5-(hydroxymethyl)uracil, 5-aminouracil pseudouracil dihydrouracil 5-fluorouracil, 5-fluorocytosine, 5-chlorouracil, 5-chlorocytosine, 5-bromouracil, 5-bromocytosine, 2,4-diaminopurine, 8-aza-purine, 7-deaza-7-substituted purine and 7-deaza-8-substituted purine;
f) the conjugation to a molecule which influences the property of the oligonucleotide, wherein the molecule which influences the property of the oligonucleotide is selected from polylysine, intercalating agents, fluorescent agents, crosslinking agents, lipophilic molecules, lipids, steroids, vitamins, poly-or oligoethylene glycol preferably linked to the oligonucleotide via a phosphate group, a (C₁₂-C₁₈)-alkyl phosphate diester and O-CH₂-CH(OH)-O-(C₁₂-C₁₈)-alkyl groups;
g) the conjugation to a 2'5'-linked oligoadenylate, preferably via an appropriate linker molecule, wherein the 2'5'-linked oligoadenylate is selected from 2'5'-linked triadenylate, 2'5'-linked tetraadenylate, 2'5'-linked pentaadenylate, 2'5'-linked hexaadenylat and 2'5'-linked heptaadenylat molecules and derivatives thereof; and
h) the introduction of a 3'-3' and/or a 5'-5' inversion at the 3' and/or the 5' end of the oligonucleotide.

8. A method of making an oligonucleotide as claimed in one or more of claims 1 to 7 by condensing suitably protected monomers on a solid support.

9. The use of an oligonucleotide as claimed in one or more of claims 1 to 7 for inhibiting the expression of VEGF.

10. A method of inhibiting the expression of VEGF, wherein an oligonucleotide as claimed in one or more of claims 1 to 7 is brought into contact with a VEGF encoding nucleic acid.

11. The use of an oligonucleotide as claimed in one or more of claims 1 to 7 for preparing a pharmaceutical composition.

12. A method of making a pharmaceutical composition by mixing one or more oligonucleotides as claimed in one or more of claims 1 to 7 with a physiologically acceptable exipient and optionally additional substances.

13. A pharmaceutical composition which comprises at least one oligonucleotide as claimed in one or more of claims 1 to 7.

14. The use of a pharmaceutical composition which comprises at least one oligonucleotide as claimed in claims 1 to 7 for the treatment of diseases, which are associated with abnormal vascular permeability, cell proliferation, cell permeation, angiogenesis, neovascularization, tumor cell growth and/or metastasis.

15. The use of a pharmaceutical composition which comprises at least one oligonucleotide as claimed in one or more of claims 1 to 7, in combination with other pharmaceuticals and/or other therapeutic methods.


SEQUENCE LISTING

(1) GENERAL INFORMATION:

(i) APPLICANT:
(A) NAME: Hoechst Marion Roussel Deutschland GmbH
(B) STREET: -
(C) CITY: Frankfurt
(D) STATE: -
(E) COUNTRY: Germany
(F) POSTAL CODE (ZIP): 65926
(G) TELEPHONE: 069-305-7072
(H) TELEFAX: 069-35-7175
(I) TELEX: -

(ii) TITLE OF INVENTION: Short Oligonucleotides for the Inhibition
of VEGF Expression

(iii) NUMBER OF SEQUENCES: 93

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..22

(xi) SEQUENCE DESCRIPTION: SEQ ID NO: 1:

CCCGGCCCCG GTCGGGCCTC CG 22

(2) INFORMATION FOR SEQ ID NO: 2:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 2:
CGGGCCTCCG AAACC 15

(2) INFORMATION FOR SEQ ID NO: 3:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..21

(xi) SEQUENCE DESCRIPTION: SEQ ID NO: 3:

GCTCTACCTC CACCATGCCA A 21

(2) INFORMATION FOR SEQ ID NO: 4:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..24

(xi) SEQUENCE DESCRIPTION: SEQ ID NO: 4:

GTGGTCCCAG GCTGCACCCA TGGC 24

(2) INFORMATION FOR SEQ ID NO: 5:

(i) SEQUENCE CHARACTERISTICS:
(A) LENGTH: 16 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..16

(xi) SEQUENCE DESCRIPTION: SEQ ID NO: 5:

CATCTTCAAG CCATCC 16

(2) INFORMATION FOR SEQ ID NO: 6:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 6:

TGCGGGGGCT GCTGC 15

(2) INFORMATION FOR SEQ ID NO: 7:

(i) SEQUENCE CHARACTERISTICS:
(A) LENGTH: 22 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..22

(xi) SEQUENCE DESCRIPTION: SEQ ID NO: 7:

GGGCCGGGGC CAGCCCGGAG GC 22

(2) INFORMATION FOR SEQ ID NO: 8:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 8:

GCCCGGAGGC TTTGG 15

(2) INFORMATION FOR SEQ ID NO: 9:

(i) SEQUENCE CHARACTERISTICS:
(A) LENGTH: 21 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..21

(xi) SEQUENCE DESCRIPTION: SEQ ID NO: 9:

CGAGATGGAG GTGGTACGGT T 21

(2) INFORMATION FOR SEQ ID NO: 10:

(i) SEQUENCE CHARACTERISTICS:
(A) LENGTH: 24 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..24

(xi) SEQUENCE DESCRIPTION: SEQ ID NO: 10:

CACCAGGGTC CGACGTGGGT ACCG 24

(2) INFORMATION FOR SEQ ID NO: 11:

(i) SEQUENCE CHARACTERISTICS:
(A) LENGTH: 16 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..16

(xi) SEQUENCE DESCRIPTION: SEQ ID NO: 11:

GTAGAAGTTC GGTAGG 16

(2) INFORMATION FOR SEQ ID NO: 12:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 12:

ACGCCCCCGA CGACG 15

(2) INFORMATION FOR SEQ ID NO: 13:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 13:

GGCCAGCCCG GA 12

(2) INFORMATION FOR SEQ ID NO: 14:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 14:

CCAGCCCGGA GG 12

(2) INFORMATION FOR SEQ ID NO: 15:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 15:

GCCCGGAGGC TT 12

(2) INFORMATION FOR SEQ ID NO: 16:

(i) SEQUENCE CHARACTERISTICS:
(A) LENGTH: 12 base pairs

(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 16:

CGGAGGCTTT GG 12

(2) INFORMATION FOR SEQ ID NO: 17:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 17:

GGCTTTGGTA CT 12

(2) INFORMATION FOR SEQ ID NO: 18:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 18:

TTGGTACTTG AA                                                          12

(2) INFORMATION FOR SEQ ID NO: 19:

     (i) SEQUENCE CHARACTERISTICS:
          (A) LENGTH: 12 base pairs
          (B) TYPE: nucleic acid
          (C) STRANDEDNESS: single
          (D) TOPOLOGY: linear
          (E) This sequence is written in 3´→ 5´ direction

    (ii) MOLECULE TYPE: DNA (genomic)

    (ix) FEATURE:
          (A) NAME/KEY: exon
          (B) LOCATION:1..12

    (xi) SEQUENCE DESCRIPTION: SEQ ID NO: 19:

GGTACTTGAA AG                                                          12

(2) INFORMATION FOR SEQ ID NO: 20:

     (i) SEQUENCE CHARACTERISTICS:
          (A) LENGTH: 12 base pairs
          (B) TYPE: nucleic acid
          (C) STRANDEDNESS: single
          (D) TOPOLOGY: linear
          (E) This sequence is written in 3´→ 5´ direction

    (ii) MOLECULE TYPE: DNA (genomic)

    (ix) FEATURE:
          (A) NAME/KEY: exon
          (B) LOCATION:1..12

    (xi) SEQUENCE DESCRIPTION: SEQ ID NO: 20:

CTTGAAAGAC GA                                                          12

(2) INFORMATION FOR SEQ ID NO: 21:

     (i) SEQUENCE CHARACTERISTICS:
          (A) LENGTH: 12 base pairs
          (B) TYPE: nucleic acid
          (C) STRANDEDNESS: single
          (D) TOPOLOGY: linear
          (E) This sequence is written in 3´→ 5´ direction

    (ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 21:

GAAAGACGAC AG 12

(2) INFORMATION FOR SEQ ID NO: 22:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 22:

GACGACAGAA CC 12

(2) INFORMATION FOR SEQ ID NO: 23:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 23:

GACAGAACCC AC 12

(2) INFORMATION FOR SEQ ID NO: 24:

(i) SEQUENCE CHARACTERISTICS:
(A) LENGTH: 12 base pairs
(B) TYPE: nucleic acid

(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 24:

GAACCCACGT AA 12

(2) INFORMATION FOR SEQ ID NO: 25:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 25:

CGACGAGATG GA 12

(2) INFORMATION FOR SEQ ID NO: 26:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 26:

CGAGATGGAG GT 12

(2) INFORMATION FOR SEQ ID NO: 27:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 27:

GATGGAGGTG GT 12

(2) INFORMATION FOR SEQ ID NO: 28:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 28:

GGAGGTGGTA CG 12

(2) INFORMATION FOR SEQ ID NO: 29:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon

(B) LOCATION:1..12

(xi) SEQUENCE DESCRIPTION: SEQ ID NO: 29:

GGTGGTACGG TT 12

(2) INFORMATION FOR SEQ ID NO: 30:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 30:

GGTACGGTTC AC 12

(2) INFORMATION FOR SEQ ID NO: 31:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 31:

ACGGTTCACC AG 12

(2) INFORMATION FOR SEQ ID NO: 32:

(i) SEQUENCE CHARACTERISTICS:
(A) LENGTH: 12 base pairs
(B) TYPE: nucleic acid
(C) STRANDEDNESS: single
(D) TOPOLOGY: linear

(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 32:

GGTTCACCAG GG 12

(2) INFORMATION FOR SEQ ID NO: 33:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 33:

CACCAGGGTC CG 12

(2) INFORMATION FOR SEQ ID NO: 34:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 34:

CCAGGGTCCG AC 12

(2) INFORMATION FOR SEQ ID NO: 35:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 35:

AGGGTCCGAC GT 12

(2) INFORMATION FOR SEQ ID NO: 36:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 36:

GGGTCCGACG TG 12

(2) INFORMATION FOR SEQ ID NO: 37:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 37:

GGTCCGACGT GG 12

(2) INFORMATION FOR SEQ ID NO: 38:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 38:

CCGACGTGGG TA 12

(2) INFORMATION FOR SEQ ID NO: 39:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 39:

CCACTTCAAG TA 12

(2) INFORMATION FOR SEQ ID NO: 40:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 40:

CTTCAAGTAC CT 12

(2) INFORMATION FOR SEQ ID NO: 41:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 41:

CAAGTACCTA CA 12

(2) INFORMATION FOR SEQ ID NO: 42:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 42:

GTACCTACAG AT 12

(2) INFORMATION FOR SEQ ID NO: 43:

(i) SEQUENCE CHARACTERISTICS:

(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 43:

ACCTACAGAT AG 12

(2) INFORMATION FOR SEQ ID NO: 44:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 44:

CTACAGATAG TC 12

(2) INFORMATION FOR SEQ ID NO: 45:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 45:

CAGATAGTCG CG 12

(2) INFORMATION FOR SEQ ID NO: 46:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 46:

GATAGTCGCG TC 12

(2) INFORMATION FOR SEQ ID NO: 47:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 47:

GTCGCGTCGA TG 12

(2) INFORMATION FOR SEQ ID NO: 48:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 48:

CGCGTCGATG AC 12

(2) INFORMATION FOR SEQ ID NO: 49:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 49:

CGTCGATGAC GG 12

(2) INFORMATION FOR SEQ ID NO: 50:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 50:

CGATGACGGT AG 12

(2) INFORMATION FOR SEQ ID NO: 51:

(i) SEQUENCE CHARACTERISTICS:
(A) LENGTH: 12 base pairs

(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 51:

GCTCATGTAG AA 12

(2) INFORMATION FOR SEQ ID NO: 52:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 52:

GTAGAAGTTC GG 12

(2) INFORMATION FOR SEQ ID NO: 53:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 53:

GAAGTTCGGT AG                                                          12

(2) INFORMATION FOR SEQ ID NO: 54:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 54:

CGGTAGGACA CA                                                          12

(2) INFORMATION FOR SEQ ID NO: 55:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 55:

ACGCCCCCGA CG                                                          12

(2) INFORMATION FOR SEQ ID NO: 56:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:

(A) NAME/KEY: exon
(B) LOCATION:1..12

(xi) SEQUENCE DESCRIPTION: SEQ ID NO: 56:

CCCCCGACGA CG 12

(2) INFORMATION FOR SEQ ID NO: 57:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 57:

CGACGTTACT GC 12

(2) INFORMATION FOR SEQ ID NO: 58:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 58:

CTCCCGGACC TC 12

(2) INFORMATION FOR SEQ ID NO: 59:

(i) SEQUENCE CHARACTERISTICS:
(A) LENGTH: 12 base pairs
(B) TYPE: nucleic acid
(C) STRANDEDNESS: single

(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 59:

CGGACCTCAC AC 12

(2) INFORMATION FOR SEQ ID NO: 60:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 60:

GACCTCACAC AC 12

(2) INFORMATION FOR SEQ ID NO: 61:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 61:

GTGGTACGTC TA 12

(2) INFORMATION FOR SEQ ID NO: 62:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 62:

GGTACGTCTA AT 12

(2) INFORMATION FOR SEQ ID NO: 63:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 63:

CGTCTAATAC GC 12

(2) INFORMATION FOR SEQ ID NO: 64:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon

(B) LOCATION:1..12

(xi) SEQUENCE DESCRIPTION: SEQ ID NO: 64:

CTAATACGCC TA 12

(2) INFORMATION FOR SEQ ID NO: 65:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 65:

CGCCTAGTTT GG 12

(2) INFORMATION FOR SEQ ID NO: 66:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 66:

AGTTTGGAGT GG 12

(2) INFORMATION FOR SEQ ID NO: 67:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 67:

GATGTCGTGT TG 12

(2) INFORMATION FOR SEQ ID NO: 68:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 68:

CGTGTTGTTT AC 12

(2) INFORMATION FOR SEQ ID NO: 69:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 69:

CACTTACGTC TG 12

(2) INFORMATION FOR SEQ ID NO: 70:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 70:

CTTACGTCTG GT 12

(2) INFORMATION FOR SEQ ID NO: 71:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 71:

CGTCTGGTTT CT 12

(2) INFORMATION FOR SEQ ID NO: 72:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 72:

CTGGTTTCTT TC 12

(2) INFORMATION FOR SEQ ID NO: 73:

(i) SEQUENCE CHARACTERISTICS:
(A) LENGTH: 13 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..13

(xi) SEQUENCE DESCRIPTION: SEQ ID NO: 73:

GGAGGTGGTA CGG 13

(2) INFORMATION FOR SEQ ID NO: 74:

(i) SEQUENCE CHARACTERISTICS:
(A) LENGTH: 13 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..13

(xi) SEQUENCE DESCRIPTION: SEQ ID NO: 74:

GGGTCCGACG TGG 13

(2) INFORMATION FOR SEQ ID NO: 75:

(i) SEQUENCE CHARACTERISTICS:
(A) LENGTH: 13 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..13

(xi) SEQUENCE DESCRIPTION: SEQ ID NO: 75:

GCCCCCGACG ACG 13

(2) INFORMATION FOR SEQ ID NO: 76:

(i) SEQUENCE CHARACTERISTICS:
(A) LENGTH: 14 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..14

(xi) SEQUENCE DESCRIPTION: SEQ ID NO: 76:

CCCGGAGGCT TTGG 14

(2) INFORMATION FOR SEQ ID NO: 77:

(i) SEQUENCE CHARACTERISTICS:
(A) LENGTH: 14 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..14

(xi) SEQUENCE DESCRIPTION: SEQ ID NO: 77:

CGAGATGGAG GTGG 14

(2) INFORMATION FOR SEQ ID NO: 78:

(i) SEQUENCE CHARACTERISTICS:
(A) LENGTH: 14 base pairs

(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..14

(xi) SEQUENCE DESCRIPTION: SEQ ID NO: 78:

GGGTCCGACG TGGG 14

(2) INFORMATION FOR SEQ ID NO: 79:

(i) SEQUENCE CHARACTERISTICS:
(A) LENGTH: 14 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..14

(xi) SEQUENCE DESCRIPTION: SEQ ID NO: 79:

CGCCCCCGAC GACG 14

(2) INFORMATION FOR SEQ ID NO: 80:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 80:

GGGCCGGGGC CAGCC 15

(2) INFORMATION FOR SEQ ID NO: 81:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 81:

CCGGGGCCAG CCCGG 15

(2) INFORMATION FOR SEQ ID NO: 82:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 82:

GGCCGGGGCC AGCCC 15

(2) INFORMATION FOR SEQ ID NO: 83:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 83:

CCCCGGAGGC TTTGG 15

(2) INFORMATION FOR SEQ ID NO: 84:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 84:

ATGGAGGTGG TACGG 15

(2) INFORMATION FOR SEQ ID NO: 85:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 85:

GGAGGTGGTA CGGTT 15

(2) INFORMATION FOR SEQ ID NO: 86:

(i) SEQUENCE CHARACTERISTICS:
(A) LENGTH: 15 base pairs
(B) TYPE: nucleic acid
(C) STRANDEDNESS: single

(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 86:

CCAGGGTCCG ACGTG 15

(2) INFORMATION FOR SEQ ID NO: 87:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 87:

GTAGAAGTTC GGTAG 15

(2) INFORMATION FOR SEQ ID NO: 88:

(i) SEQUENCE CHARACTERISTICS:
(A) LENGTH: 15 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..15

(xi) SEQUENCE DESCRIPTION: SEQ ID NO: 88:

TAGAAGTTCG GTAGG 15

(2) INFORMATION FOR SEQ ID NO: 89:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 89:

GGAGTGGGTA CG 12

(2) INFORMATION FOR SEQ ID NO: 90:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 90:

GGCGTGGGTA AG 12

(2) INFORMATION FOR SEQ ID NO: 91:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon

(B) LOCATION:1..12

(xi) SEQUENCE DESCRIPTION: SEQ ID NO: 91:

GGGTCCAGCG TG 12

(2) INFORMATION FOR SEQ ID NO: 92:

(i) SEQUENCE CHARACTERISTICS:
(A) LENGTH: 12 base pairs
(B) TYPE: nucleic acid
(C) STRANDEDNESS: single
(D) TOPOLOGY: linear
(E) This sequence is written in 3´→ 5´ direction

(ii) MOLECULE TYPE: DNA (genomic)

(ix) FEATURE:
(A) NAME/KEY: exon
(B) LOCATION:1..12

(xi) SEQUENCE DESCRIPTION: SEQ ID NO: 92:

GGGCCCAGTG TG 12

(2) INFORMATION FOR SEQ ID NO: 93:

(i) SEQUENCE CHARACTERISTICS:
(A) LENGTH: 480 base pairs
(B) TYPE: nucleic acid
(C) STRANDEDNESS: single
(D) TOPOLOGY: linear

(ii) MOLECULE TYPE: DNA (genomic)

(ix) FEATURE:
(A) NAME/KEY: exon
(B) LOCATION:1..480

(xi) SEQUENCE DESCRIPTION: SEQ ID NO: 93:

CAGTGTGCTG GCGGCCCGGC GCGAGCCGGC CCGGCCCCGG TCGGGCCTCC GAAACCATGA 60

ACTTTCTGCT GTCTTGGGTG CATTGGAGCC TCGCCTTGCT GCTCTACCTC CACCATGCCA 120

AGTGGTCCCA GGCTGCACCC ATGGCAGAAG GAGGAGGGCA GAATCATCAC GAAGTGGTGA 180

AGTTCATGGA TGTCTATCAG CGCAGCTACT GCCATCCAAT CGAGACCCTG GTGGACATCT 240

TCCAGGAGTA CCCTGATGAG ATCGAGTACA TCTTCAAGCC ATCCTGTGTG CCCCTGATGC 300

GATGCGGGGG CTGCTGCAAT GACGAGGGCC TGGAGTGTGT GCCCACTGAG GAGTCCAACA 360

TCACCATGCA GATTATGCGG ATCAAACCTC ACCAAGGCCA GCACATAGGA GAGATGAGCT 420

TCCTACAGCA CAACAAATGT GAATGCAGAC CAAAGAAAGA TAGAGCAAGA CAAGAAAATC 480